# EUROPEAN PATENT APPLICATION

(11) **EP 2 316 928 A1**
(43) Date of publication of application: **04.05.2011**
(21) Application number: 09013639.1
(22) Date of filing: 29.10.2009
(51) Int. Cl.: C12N 9/16, C12N 15/82

(54) **Improvement of photosynthesis rate**

(71) Applicant: Ludwig-Maximilians-Universität München, 80359 München (DE)
(72) Inventor: Leister, Dario, 81373 München (DE); Pribil, Mathias, 81539 München (DE)
(74) Representative: Engelhard, Markus

(57) **Abstract**

The invention relates to methods of increasing the photosynthesis rate of a plant cell and/or the production of biomass in a plant, as well as to plant cells and plants with increased photosynthesis rate/production of biomass. The present invention also relates to a phosphatase of the light-harvesting complex of photosystem II, to nucleic acids coding for such phosphatase, and to mutants of such nucleic acids. Moreover, the present invention relates to plant cells and plants, wherein the activity of said phosphatase is inhibited; in particular the present invention relates to plant cells and plants comprising the mutant nucleic acids

SEQUENCE LISTING

<110>  Ludwig-Maximilians-Universität München

<120>  Improvement of photosynthesis rate

<130>  FB22357

<160>  22

<170>  PatentIn version 3.5

<210>  1
<211>  388
<212>  PRT
<213>  Arabidopsis thaliana

<400>  1

Met Ala Leu Leu Arg Pro His Leu His Arg Phe His Ser Asn Thr Leu
1               5                   10                  15

Arg His Ser Ala Tyr Pro Ser Ala Asp Ala Gly Gly Gly Leu Val Val
            20                  25                  30

Tyr Pro Thr Tyr Gly Arg His Arg Cys Ser Ala Ile Ala Ile Asp Ala
        35                  40                  45

Pro Ser Ser Leu Thr Gly Val Thr Pro Ile Arg Trp Gly Tyr Thr Ser
    50                  55                  60

Val Gln Gly Phe Arg Asp Glu Met Glu Asp Asp Ile Val Ile Arg Ser
65                  70                  75                  80

Asp Ala Val Asp Ser Phe Ser Tyr Ala Ala Val Phe Asp Gly His Ala
                85                  90                  95

Gly Ser Ser Ser Val Lys Phe Leu Arg Glu Glu Leu Tyr Lys Glu Cys
            100                 105                 110

Val Gly Ala Leu Gln Ala Gly Ser Leu Leu Asn Gly Gly Asp Phe Ala
        115                 120                 125

Ala Ile Lys Glu Ala Leu Ile Lys Ala Phe Glu Ser Val Asp Arg Asn
    130                 135                 140

Leu Leu Lys Trp Leu Glu Ala Asn Gly Asp Glu Glu Asp Glu Ser Gly
145                 150                 155                 160

Ser Thr Ala Thr Val Met Ile Ile Arg Asn Asp Val Ser Phe Ile Ala
                165                 170                 175

His Ile Gly Asp Ser Cys Ala Val Leu Ser Arg Ser Gly Gln Ile Glu
                180                 185                 190

Glu Leu Thr Asp Tyr His Arg Pro Tyr Gly Ser Ser Arg Ala Ala Ile
        195                 200                 205

Gln Glu Val Lys Arg Val Lys Glu Ala Gly Gly Trp Ile Val Asn Gly
    210                 215                 220

Arg Ile Cys Gly Asp Ile Ala Val Ser Arg Ala Phe Gly Asp Ile Arg
225                 230                 235                 240

Phe Lys Thr Lys Lys Asn Asp Met Leu Lys Lys Gly Val Asp Glu Gly
                245                 250                 255

Arg Trp Ser Glu Lys Phe Val Ser Arg Ile Glu Phe Lys Gly Asp Met
                260                 265                 270

Val Val Ala Thr Pro Asp Ile Phe Gln Val Pro Leu Thr Ser Asp Val
        275                 280                 285

Glu Phe Ile Ile Leu Ala Ser Asp Gly Leu Trp Asp Tyr Met Lys Ser
    290                 295                 300

Ser Asp Val Val Ser Tyr Val Arg Asp Gln Leu Arg Lys His Gly Asn
305                 310                 315                 320

Val Gln Leu Ala Cys Glu Ser Leu Ala Gln Val Ala Leu Asp Arg Arg
                325                 330                 335

Ser Gln Asp Asn Ile Ser Ile Ile Ile Ala Asp Leu Gly Arg Thr Glu
                340                 345                 350

Trp Lys Asn Leu Pro Ala Gln Arg Gln Asn Val Val Val Glu Leu Val
        355                 360                 365

Gln Ala Ala Thr Thr Ile Gly Leu Val Thr Val Gly Ile Trp Met Ser
    370                 375                 380

Ser His Leu Ser
385

<210>  2
<211>  386
<212>  PRT
<213>  Vitis vinifera

<400>  2

Met Ala Leu Leu Ser Gln Gln Leu Gln Arg Phe Phe Leu Thr Thr Leu
1               5                   10                  15

Asn Cys Thr Ser Asn Leu Lys Ala Thr Lys Lys Asn Asn Ser Val Ala
            20                  25                  30

Leu Lys Glu Arg Ala Arg Cys Cys Ser Ala Ile Ala Ile Asp Ala Pro
        35                  40                  45

Ser Phe Ser Gly Val Ala Gly Ile Arg Trp Gly Leu Cys Arg Leu Gln
    50                  55                  60

Gly Pro Arg Glu Glu Met Glu Asp Glu Ala Val Val Arg Ser Asp Gly
65                  70                  75                  80

Leu Asp Gly Phe Ser Phe Ala Ala Val Phe Asp Gly His Ala Gly Phe
                85                  90                  95

Ser Ser Val Lys Phe Leu Arg Asp Glu Leu Tyr Lys Asp Cys Val Ala
            100                 105                 110

Ala Leu Gln Gly Gly Leu Leu Leu Ser Gly Lys Asn Phe Asn Ile Ile
        115                 120                 125

Arg Glu Ala Leu Glu Lys Ala Phe Glu Ser Ala Asp Ala Lys Leu Leu
    130                 135                 140

Asn Trp Leu Glu Thr Thr Gly Glu Asp Val Glu Ser Gly Ser Thr Ala
145                 150                 155                 160

Thr Val Leu Leu Ile Gly Asp Asp Met Val Phe Ile Ser His Val Gly
                165                 170                 175

Asp Ser Cys Val Val Leu Ser Arg Ser Gly Lys Ala Glu Glu Leu Thr
            180                 185                 190

Asn Pro His Arg Pro Tyr Gly Ser Asn Lys Ser Ser Leu Glu Glu Ile
        195                 200                 205

Arg Arg Ile Arg Glu Ala Gly Gly Trp Ile Val Asn Gly Arg Ile Cys
    210                 215                 220

Gly Asp Ile Ala Val Ser Arg Ser Phe Gly Asp Met Arg Phe Lys Thr
225                 230                 235                 240

Lys Lys Asn Glu Met Leu Glu Lys Gly Leu Glu Glu Gly Arg Trp Ser
                245                 250                 255

Gln Lys Phe Val Ser Arg Val Gln Phe Thr Gly Asp Leu Val Val Ala
            260                 265                 270

Ser Pro Asp Val Phe Gln Val Ala Leu Gly Ser Asp Ala Glu Phe Leu
        275                 280                 285

Leu Leu Ala Ser Asp Gly Leu Trp Asp Tyr Met Asn Ser Ser Glu Ala
    290                 295                 300

Val Thr Phe Val Arg Asn Glu Leu Arg Gln His Gly Asp Val Gln Val
305                 310                 315                 320

Ala Ser Glu Ala Leu Ala Arg Ala Ala Leu Asp Arg Arg Thr Gln Asp
                325                 330                 335

Asn Val Ser Ile Ile Ile Ala Asp Leu Gly Arg Thr Asp Trp Gln Ser
            340                 345                 350

Leu Pro Leu Gln Gln Gln Asn Leu Phe Leu Glu Leu Val Gln Ala Leu
        355                 360                 365

Gly Thr Ile Gly Ile Val Thr Leu Gly Ile Trp Met Ser Ser Gln Phe
    370                 375                 380

Ser Leu
385

<210>  3
<211>  389
<212>  PRT
<213>  Oryza sativa

<400>  3

Met Ala Leu Leu Ser Pro Arg Val Pro Arg Leu Pro Leu Ala Ser Ala
1               5                   10                  15

Ser Ala Ala Gly Ala Gly Leu Arg Cys Cys Val Ser Gly Gly Arg Ala
            20                  25                  30

Gly Ser Ala Ala Trp Cys His Ala Ser Ala Ala Gly Ser Val Ala Ser
        35                  40                  45

Ser Ser Ser Glu Leu Glu Ala Ile Arg Trp Gly Thr Ala Lys Leu Gln
    50                  55                  60

Gly Ala Arg Asp Glu Met Glu Asp Glu Val Val Leu Arg Pro Gly Ser
65                  70                  75                  80

Leu Leu Asp Gly Phe Ser Phe Ala Ala Val Phe Asp Gly His Ala Gly
                85                  90                  95

Phe Ser Ala Val Glu Phe Leu Arg Asp Glu Leu Tyr Lys Glu Cys Ala
            100                 105                 110

Ala Ala Leu Asp Gly Gly Ala Val Leu Ser Thr Lys Asn Leu Asp Ala
        115                 120                 125

Ile Thr Asp Ser Ile Gln Arg Ala Phe Ala Thr Val Asp Ala Asn Leu
    130                 135                 140

Ser Thr Trp Leu Glu Gln Met Asp Lys Glu Asp Glu Ser Gly Ala Thr
145                 150                 155                 160

Ala Thr Ala Met Phe Leu Arg Asn Asp Val Leu Val Val Ser His Ile
                165                 170                 175

Gly Asp Ser Cys Leu Gln Val Val Ser Arg Gly Gly Arg Pro Gln Ala
            180                 185                 190

Val Thr Asn Phe His Arg Pro Tyr Gly Asn Lys Lys Ala Ser Leu Glu
        195                 200                 205

Glu Val Lys Arg Ile Arg Ala Ala Gly Gly Trp Ile Val Asp Gly Arg
    210                 215                 220

Ile Cys Gly Glu Ile Ser Val Ser Arg Ala Phe Gly Asp Ile Arg Phe
225                 230                 235                 240

Lys Thr Arg Lys Asn Glu Met Leu Val Lys Gly Val Lys Glu Gly Arg
                245                 250                 255

Trp Thr Glu Lys Phe Ile Ser Arg Ile Asn Phe Lys Gly Asp Leu Ile
            260                 265                 270

Val Ser Ser Pro Asp Val Ser Leu Val Glu Leu Gly Pro Asp Val Glu
        275                 280                 285

Phe Val Leu Leu Ala Thr Asp Gly Leu Trp Asp Tyr Ile Lys Ser Ser
    290                 295                 300

Glu Ala Val Ala Leu Val Arg Asp Gln Leu Arg Gln His Gly Asp Val
305                 310                 315                 320

Gln Val Ala Cys Glu Ala Leu Gly Gln Ile Ala Leu Asp Arg Arg Ser
                325                 330                 335

Gln Asp Asn Ile Ser Ile Val Ile Ala Asp Leu Gly Arg Thr Asn Trp
            340                 345                 350

Lys Glu Leu Pro Ala Gln Arg Pro Asn Leu Phe Leu Glu Leu Thr Gln
        355                 360                 365

Ala Val Ala Thr Val Gly Ala Val Ser Leu Gly Ile Tyr Ile Ser Ser
    370                 375                 380

Leu Leu Ala Leu Gln
385

<210>  4
<211>  393
<212>  PRT
<213>  Hordeum vulgare

<400>  4

Glu Phe Gly Thr Arg Leu Ser Pro Arg Val Pro Arg Leu Pro Leu Ala
1               5                   10                  15

Ala His Cys Ala Gly Ala Gly Ala Gly Ile Leu Arg Cys Cys Gly Arg
            20                  25                  30

Leu Ala Ala Ser Ala Ala Arg Cys Gln Val Thr Ala Ser Gly Ser Val
        35                  40                  45

Ala Ala Gly Ser Ser Ser Ser Ser Glu Leu Glu Ala Ile Arg Trp Gly
    50                  55                  60

Ser Ala Lys Leu Gln Gly Ala Arg Glu Glu Met Glu Asp Glu Val Val
65                  70                  75                  80

Leu Arg Pro Glu Ser Leu Leu His Gly Phe Ser Phe Ala Ala Val Leu
                85                  90                  95

Asp Gly His Ala Gly Phe Ser Thr Val Gln Phe Leu Arg Asp Glu Leu
            100                 105                 110

Phe Lys Glu Cys Ala Ala Ala Leu Asp Gly Gly Ala Val Leu Asn Thr
        115                 120                 125

Lys Asn Leu Glu Ala Ile Thr Asp Ser Ile Arg Arg Ala Phe Ala Thr
    130                 135                 140

Val Asp Ala Asn Leu Ser Thr Trp Leu Gln Gln Met Asp Lys Glu Asp
145                 150                 155                 160

Asp Ser Gly Ala Thr Ala Thr Ala Leu Phe Leu Arg Asn Asp Val Leu
                165                 170                 175

Val Val Ser His Ile Gly Asp Ser Cys Leu Val Ile Ser Arg Gly Gly
            180                 185                 190

Arg Pro Gln Ser Leu Thr Asn Phe His Arg Pro Tyr Gly Asn Asn Lys
        195                 200                 205

Thr Ser Leu Glu Glu Val Lys Arg Ile Arg Ala Ala Gly Gly Trp Ile
    210                 215                 220

His Asp Gly Arg Ile Cys Gly Asp Ile Ser Val Ser Arg Ala Phe Gly
225                 230                 235                 240

Asp Ile Arg Phe Lys Thr Arg Lys Asn Glu Met Leu Val Lys Gly Val
                245                 250                 255

Lys Glu Gly Arg Trp Thr Glu Lys Phe Val Ser Arg Ile Lys Phe Asn
            260                 265                 270

Gly Asp Leu Ile Ile Ser Ser Pro Asp Val Ser Leu Val Glu Leu Gly
        275                 280                 285

Pro Asp Ala Glu Phe Val Leu Val Ala Thr Asp Gly Leu Trp Asp Tyr
    290                 295                 300

Ile Lys Ser Thr Glu Ala Val Ala Phe Val Arg Asp Gln Leu Arg Gln
305                 310                 315                 320

His Gly Asp Val Gln Arg Ala Cys Glu Ala Leu Gly Glu Lys Ala Leu
                325                 330                 335

Asp Arg Arg Ser Gln Asp Asn Ile Ser Ile Val Ile Ala Asp Leu Gly
            340                 345                 350

Arg Thr Asn Trp Gln Glu Leu Ala Val Pro Arg Pro Asn Val Leu Leu
        355                 360                 365

Glu Leu Ser Gln Ala Ile Ala Thr Val Gly Ala Val Ser Val Gly Ile
    370                 375                 380

Trp Ile Ser Trp Leu Leu Thr Leu Gln
385                 390

<210>  5

<211>  394
<212>  PRT
<213>  Lycopersicon esculentum

<400>  5

Met Ala Leu Ser Ser Pro His Leu Gln Lys Val Leu Leu Asn Lys Ile
1               5                   10                  15

Cys Ser Tyr Lys Ile Thr Ser Lys Lys Ser Ile Ile Gln Lys Pro Leu
            20                  25                  30

Gly Gly Gly Gly Gly Gly Gly Gly Glu Gly Arg Gly Arg Cys Cys Ser
        35                  40                  45

Ala Ile Ala Ile Asp Ala Pro Ala Pro Phe Thr Ser Val Ser Gly Ile
    50                  55                  60

Arg Trp Gly Ser Thr Met Val Gln Gly Pro Arg Glu Glu Met Glu Asp
65                  70                  75                  80

Asp Ala Val Ile Val Gln Ser Asp Asp Leu Asp Gly Phe Thr Tyr Ala
                85                  90                  95

Ala Val Phe Asp Gly His Ala Gly Phe Ser Ser Val Lys Phe Leu Arg
            100                 105                 110

Glu Glu Leu Tyr Lys Glu Cys Val Leu Ala Leu Gln Gly Gly Leu Leu
        115                 120                 125

Leu Asn Arg Lys Asp Leu Asn Ala Ile Arg Lys Ala Leu Gln Glu Ala
    130                 135                 140

Phe Glu Asn Ala Asp Arg Lys Leu Leu Asn Trp Leu Glu Ser Ser Glu
145                 150                 155                 160

Lys Glu Asp Glu Ser Gly Ala Thr Ala Thr Ala Leu Phe Val Gly Asn
                165                 170                 175

Asp Thr Leu Ile Ile Ala His Val Gly Asp Ser Ser Val Val Leu Ser
            180                 185                 190

Arg Ser Gly Lys Thr Glu Ile Leu Thr Asn Ser His Arg Pro Tyr Gly
        195                 200                 205

Asn Asn Lys Val Ser Leu Gln Glu Ile Arg Arg Ile Asn Glu Ala Gly
    210                 215                 220

Gly Trp Ile Val Asn Gly Arg Ile Cys Asn Asp Ile Ser Val Ser Arg

225                 230                 235                 240

Ala Phe Gly Asp Met Arg Phe Lys Thr Lys Lys Lys Glu Met Leu Glu
                245                 250                 255

Lys Gly Val Lys Glu Arg Arg Trp Ser Glu Lys Phe Ile Ser Arg Ile
            260                 265                 270

Gln Phe Arg Gly Asp Leu Val Ile Ala Ser Pro Asp Val Leu Gln Val
        275                 280                 285

Asn Leu Gly Ser Asp Ala Glu Phe Val Leu Leu Ala Ser Asp Gly Leu
    290                 295                 300

Trp Asp Tyr Met Lys Ser Asp Glu Ile Val Asp Phe Val Arg Asn Gln
305                 310                 315                 320

Leu Arg Glu His Gly Asp Val Gln Ile Ala Cys Glu Ala Leu Ala Leu
                325                 330                 335

Leu Ala Leu Asp Arg Arg Thr Gln Asp Asn Val Ser Ile Val Ile Ala
            340                 345                 350

Asp Leu Gly Arg Thr Asp Trp Arg Asn Leu Pro Val Lys Lys Gln Asn
        355                 360                 365

Val Val Leu Glu Leu Gly Gln Ala Leu Val Thr Ile Ser Phe Val Ser
    370                 375                 380

Ile Gly Ile Trp Leu Ser Thr Met Ile Ser
385                 390

<210>  6
<211>  378
<212>  PRT
<213>  Zea mays

<400>  6

Met Ala Leu Leu Ser Pro Arg Val Pro Arg Leu Pro Pro Ser Thr Phe
1               5                   10                  15

Thr Ser Val Ala Gly Ala Arg Cys Phe Gly Pro Ala Ala Arg Cys Gln
            20                  25                  30

Ala Thr Ala Ala Gly Gly Val Ala Ala Ala Gly Pro Pro Ser Ser Glu
        35                  40                  45

Leu Glu Ala Ile Arg Trp Gly Ser Ala Lys Leu Gln Gly Ala Arg Asp

50                  55                  60

Glu Met Glu Asp Glu Val Leu Leu Arg Pro Gly Ser Leu Leu Asp Gly
65                  70                  75                  80

Phe Ser Phe Ala Ala Val Leu Asp Gly His Ala Gly Phe Ser Ala Val
                85                  90                  95

Gln Phe Leu Arg Asp Glu Leu Tyr Lys Glu Cys Ala Ala Ala Leu Asp
            100                 105                 110

Gly Gly Ala Val Leu Ser Thr Lys Asn Leu Asp Ala Ile Thr Ala Ser
        115                 120                 125

Ile Gln Arg Ala Phe Ala Ala Val Asp Ala Lys Leu Ser Thr Trp Leu
    130                 135                 140

Glu Gln Thr Asp Lys Asp Asp Ser Gly Ala Thr Ala Thr Val Met Phe
145                 150                 155                 160

Leu Arg Ser Asp Val Leu Val Val Ser His Ile Gly Asp Ser Cys Leu
                165                 170                 175

Val Ile Ser Arg Gly Gly Arg Ser Glu Ala Leu Thr Gly Ser His Arg
            180                 185                 190

Pro Tyr Gly Asn Asn Lys Thr Ser Leu Glu Glu Val Lys Arg Ile Arg
        195                 200                 205

Ala Ala Gly Gly Trp Ile Val Asp Gly Arg Ile Cys Gly Asp Ile Ser
    210                 215                 220

Val Ser Arg Ala Phe Gly Asp Ile Arg Phe Lys Thr Gln Lys Asn Glu
225                 230                 235                 240

Met Leu Val Lys Gly Val Lys Gln Gly Arg Trp Thr Asp Lys Phe Ile
                245                 250                 255

Ser Arg Ile Gln Phe Lys Asp Asp Ile Val Ile Ser Ser Pro Asp Val
            260                 265                 270

Ser Leu Val Glu Leu Gly Pro Asp Val Glu Phe Val Leu Leu Ala Asn
        275                 280                 285

Gly Leu Trp Asp Tyr Ile Lys Ser Ser Glu Ala Val Ala Phe Val Arg
    290                 295                 300

Asp Gln Leu Arg Gln His Gly Asp Val Gln Leu Ala Cys Glu Ala Leu
305                 310                 315                 320

Gly Gln Lys Ala Leu Asp Gln Arg Ser Gln Asp Asn Ile Ser Ile Val
                325                 330                 335

Ile Ala Asp Leu Gly Arg Thr Asn Trp Lys Ala Leu Pro Asp Glu Arg
            340                 345                 350

Pro Asn Leu Phe Leu Leu Ser Gln Ala Val Ala Thr Val Gly Val Val
        355                 360                 365

Ser Ile Gly Ile Trp Val Ser Ser Phe Leu
    370                 375

<210>  7
<211>  348
<212>  PRT
<213>  Triticum aestivum

<400>  7

Ile Arg Trp Gly Ser Ala Lys Leu Gln Gly Ala Arg Glu Glu Met Glu
1               5                   10                  15

Asp Glu Val Val Leu Arg Pro Asp Ser Leu Leu His Gly Phe Ser Phe
            20                  25                  30

Ala Ala Val Leu Asp Gly His Ala Gly Phe Ser Thr Val Gln Phe Leu
        35                  40                  45

Arg Asp Glu Leu Phe Lys Glu Cys Ala Ala Ala Leu Asp Gly Gly Ala
    50                  55                  60

Val Leu Asn Thr Lys Asn Leu Glu Ala Ile Thr Asp Ser Ile Arg Arg
65                  70                  75                  80

Ala Phe Ala Thr Met Asp Ala Asn Leu Ser Thr Trp Leu Gln Gln Met
                85                  90                  95

Asp Lys Glu Asp Asp Ser Gly Ala Thr Ala Thr Ala Leu Phe Leu Arg
            100                 105                 110

Asn Asp Val Leu Val Val Ser His Ile Gly Asp Ser Cys Leu Val Ile
        115                 120                 125

Ser Arg Gly Gly Arg Pro Gln Ser Leu Thr Asn Phe His Arg Pro Tyr
    130                 135                 140

Gly Asn Asn Lys Thr Ser Leu Glu Glu Val Lys Arg Ile Arg Ala Ala
145                 150                 155                 160

Gly Gly Trp Ile Arg Asp Gly Arg Val Cys Gly Asp Ile Ser Val Ser
                165                 170                 175

Arg Ala Phe Gly Asp Ile Arg Phe Lys Thr Arg Lys Asn Glu Phe Val
            180                 185                 190

Met Leu Val Lys Gly Val Asn Glu Gly Arg Trp Thr Glu Lys Phe Val
        195                 200                 205

Ser Arg Gly Arg Trp Cys Thr Val Arg Leu Phe Leu Ile Met Gln Pro
    210                 215                 220

Val Thr Cys Arg Ile Lys Phe Lys Gly Asp Leu Ile Ile Ser Ser Pro
225                 230                 235                 240

Asp Val Ser Leu Val Glu Leu Gly Pro Asp Val Glu Phe Val Leu Val
                245                 250                 255

Ala Thr Asp Gly Leu Trp Asp Tyr Ile Lys Ser Thr Glu Ala Val Ala
            260                 265                 270

Phe Val Arg Asp Gln Leu Cys Gln His Gly Asp Val Gln Val Cys Ser
        275                 280                 285

Leu Ser Leu Leu Lys Asp Arg Arg Ser Gln Asp Asn Ile Ser Ile Val
    290                 295                 300

Ile Ala Asp Leu Gly Arg Thr Asn Trp Gln Glu Leu Pro Val Pro Thr
305                 310                 315                 320

Pro Asn Val Leu Leu Glu Leu Ser Gln Ala Val Ala Thr Val Gly Ala
                325                 330                 335

Val Ser Val Gly Ile Trp Ile Ser Ser Leu Leu Thr
            340                 345

<210>  8
<211>  388
<212>  PRT
<213>  Sorghum bicolor

<400>  8

Met Ala Leu Leu Ser Pro Arg Val Pro Arg Leu Pro Pro Ser Thr Phe
1               5                   10                  15

Thr Phe Thr Phe Ala Ala Ala Ala Gly Ala Pro Cys Phe Gly Arg Ala
            20                  25                  30

Ala Arg Cys Gln Ala Thr Ala Ala Gly Gly Val Ala Ala Ala Gly Pro
        35                  40                  45

Pro Ser Ser Glu Leu Glu Ala Val Gln Trp Gly Ser Ala Lys Leu Gln
    50                  55                  60

Gly Val Arg Asp Glu Met Glu Asp Glu Ile Leu Leu Arg Pro Gly Ser
65                  70                  75                  80

Leu Leu Asp Gly Phe Ser Phe Ala Ala Val Leu Asp Gly His Ala Gly
                85                  90                  95

Phe Ser Ala Val Gln Phe Leu Arg Asp Glu Leu Tyr Lys Glu Cys Ala
            100                 105                 110

Ala Ala Leu Asp Gly Gly Ala Val Leu Ser Thr Lys Asn Leu Asp Ala
        115                 120                 125

Ile Thr Val Ser Ile Gln Arg Ala Phe Ala Ala Val Asp Ala Lys Leu
    130                 135                 140

Ser Thr Trp Leu Glu Gln Ala Asp Lys Asp Asp Asp Ser Gly Ala Thr
145                 150                 155                 160

Ala Thr Ala Met Phe Leu Arg Asn Asp Val Leu Val Val Ser His Ile
                165                 170                 175

Gly Asp Ser Cys Leu Val Ile Ser Arg Gly Gly Arg Pro Glu Ala Leu
            180                 185                 190

Thr Ser Ser His Arg Pro Tyr Gly Asn Asn Lys Thr Ser Leu Glu Glu
        195                 200                 205

Val Lys Arg Ile Arg Ala Ala Gly Gly Trp Ile Val Asp Gly Arg Ile
    210                 215                 220

Cys Gly Asp Ile Ser Val Ser Arg Ala Phe Gly Asp Ile Arg Phe Lys
225                 230                 235                 240

Thr Arg Lys Asn Glu Met Leu Val Lys Gly Val Lys Glu Gly Arg Trp
                245                 250                 255

Thr Asp Lys Phe Ile Ser Arg Ile Lys Phe Lys Asp Asp Leu Ile Ile
            260                 265                 270

Ser Ser Pro Asp Val Ser Leu Val Glu Leu Gly Pro Asp Val Glu Phe
        275                 280                 285

Val Leu Leu Ala Thr Asp Gly Leu Trp Asp Tyr Ile Lys Ser Ser Glu
    290                 295                 300

Ala Val Ala Phe Val Arg Asp Gln Leu Arg Gln His Gly Asp Val Gln
305                 310                 315                 320

Leu Ala Cys Glu Ala Leu Gly Gln Lys Ala Leu Asp Gln Arg Ser Lys
                325                 330                 335

Asp Asn Ile Ser Ile Val Ile Ala Asp Leu Gly Arg Thr Asn Trp Lys
            340                 345                 350

Ala Leu Pro Asp Glu Arg Pro Asn Met Phe Leu Glu Leu Ser Gln Ala
        355                 360                 365

Val Ala Thr Val Gly Val Val Ser Ile Gly Ile Trp Phe Ser Ser Phe
    370                 375                 380

Leu Gly Leu Gln
385

<210> 9
<211> 384
<212> PRT
<213> Populus balsamifera subsp. trichocarpa

<400> 9

Met Ala Leu Phe Arg Pro Gln Leu Glu Arg Phe Leu Ser Thr Lys Leu
1               5                   10                  15

His Cys Gly His Ser Ser Thr Lys Thr Pro Ala Lys Asn Phe Phe Phe
            20                  25                  30

Ala Thr Arg Pro Lys Ser Gln Cys Ser Ala Ile Ala Ile Asp Ala Pro
        35                  40                  45

Ser Ser Leu Thr Asp Val Ala Gly Ile Arg Trp Gly Ser Ala Ser Leu
    50                  55                  60

Gln Gly Ala Arg Glu Glu Met Glu Asp Asp Ile Ile Ile Arg Ser Asp
65                  70                  75                  80

Gly Leu Glu Gly Phe Ser Phe Ala Ala Val Phe Asp Gly His Ala Gly
                85                  90                  95

Phe Ser Ser Val Lys Phe Leu Arg Asp Glu Leu Tyr Lys Glu Cys Val
            100                 105                 110

Ala Ala Leu Gln Gly Gly Leu Leu Leu Asn Gly Lys Asp Phe Asn Ala
        115                 120                 125

Ile Arg Lys Ala Leu Glu Glu Ala Phe Glu Arg Val Asp Ala Lys Leu
    130                 135                 140

Leu Asp Trp Tyr Ala Cys Arg Asp Gly Val Gln Asp Glu Ser Gly Ser
145                 150                 155                 160

Thr Ala Thr Ala Met Phe Ile Gly Asn Glu Lys Leu Val Ile Ser His
                165                 170                 175

Ile Gly Asp Ser Ser Val Val Leu Ser Arg Ser Gly Lys Met Glu Val
            180                 185                 190

Leu Thr Asp Ala His Arg Pro Tyr Gly Ser Asn Lys Ile Ser Leu Gln
        195                 200                 205

Glu Ile Lys Arg Ile Arg Glu Ala Gly Gly Trp Ile Val Asn Gly Arg
    210                 215                 220

Ile Cys Gly Asp Ile Ala Val Ser Arg Ala Phe Gly Asp Met Arg Phe
225                 230                 235                 240

Lys Thr Lys Lys Asn Glu Met Leu Glu Lys Gly Val Lys Glu Gly Arg
                245                 250                 255

Trp Ser Glu Lys Phe Ser Ser Arg Val Gln Phe Asn Gly Asp Leu Val
            260                 265                 270

Ile Ala Ser Pro Glu Val Tyr Gln Ile Ala Phe Gly Ser Asp Ala Glu
        275                 280                 285

Phe Val Ile Leu Ala Ser Asp Gly Leu Trp Asp Tyr Met Asn Ser Leu
    290                 295                 300

Asp Ala Ala Ala Phe Val Arg Asn Gln Leu Gln Lys His Gly Asp Val
305                 310                 315                 320

Gln Leu Ala Cys Glu Glu Leu Ala Arg Lys Ala Ile Asp Arg Arg Thr
                325                 330                 335

Gln Asp Asn Val Ser Ile Ile Ile Ala Asp Leu Gly Arg Thr Asp Trp
            340                 345                 350

Gln Asn Leu Pro Leu Gln Gln Gln Asn Val Val Leu Glu Leu Gly Gln
        355                 360                 365

Ala Phe Ala Thr Ile Gly Ile Val Thr Leu Gly Ile Trp Met Ser Ser
    370                 375                 380

<210>  10
<211>  390
<212>  PRT
<213>  Ricinus communis

<400>  10

Met Ala Leu Phe Ser Pro Gln Leu Glu Arg Phe Leu Leu Ser Lys Leu
1               5                   10                  15

His Tyr Gly Ser Ser Asn Phe Lys Thr Thr Ser Thr Lys Asn Leu Asn
            20                  25                  30

Asn Phe Val Pro Val Arg Pro Ile Ser Gln Cys Ser Ala Ile Ala Ile
        35                  40                  45

Asp Ala Pro Ser Ser Leu Thr Asp Val Ala Gly Val Arg Trp Gly Ser
    50                  55                  60

Ala Ser Leu Gln Gly Ala Arg Glu Glu Met Glu Asp Tyr Ile Ile Val
65                  70                  75                  80

Arg Ser Asp Gly Leu Asp Gly Tyr Ser Phe Ala Gly Val Phe Asp Gly
                85                  90                  95

His Gly Gly Ile Ser Ser Val Glu Phe Leu Arg Asp Glu Leu Tyr Lys
            100                 105                 110

Glu Cys Val Ala Ala Leu Gln Gly Gly Leu Leu Leu Ser Gly Lys Asp
        115                 120                 125

Phe Asn Ala Thr Arg Lys Ala Leu Thr Glu Ala Phe Glu Asn Val Asp
    130                 135                 140

Lys Lys Leu Leu Asn Trp Leu Glu Thr Ile Gly Glu Glu Asp Glu Ser
145                 150                 155                 160

Gly Ser Thr Ala Thr Val Met Phe Ile Gly Asn Asn Met Leu Ile Val
                165                 170                 175

Ser His Ile Gly Asp Ser Cys Leu Val Leu Ser Arg Ser Gly Lys Ala
            180                 185                 190

Glu Val Leu Thr Glu Ser His Arg Pro Tyr Gly Ser Asn Lys Val Ser
        195                 200                 205

Leu Gln Glu Ile Lys Arg Ile Arg Glu Ala Gly Gly Trp Ile Ser Asn
    210                 215                 220

Gly Arg Ile Cys Gly Asp Ile Ala Val Ser Arg Ala Phe Gly Asp Ile
225                 230                 235                 240

Arg Phe Lys Thr Lys Lys Asn Glu Met Leu Gln Lys Gly Val Lys Glu
                245                 250                 255

Gly Arg Trp Ser Glu Lys Phe Ile Ser Arg Val Gln Phe Asn Gly Asp
            260                 265                 270

Leu Met Thr Ala Ser Pro Asp Val Phe Gln Val Ala Leu Gly Ser Asp
        275                 280                 285

Ala Glu Phe Ile Met Leu Ala Ser Asp Gly Leu Trp Asp Tyr Met Asn
    290                 295                 300

Ser Ser Asp Ala Val Ser Phe Val Arg Asn Gln Leu Arg Gln His Gly
305                 310                 315                 320

Asp Val Gln Leu Ala Cys Glu Glu Leu Ala Gln Ala Ala Leu Asp Leu
                325                 330                 335

Arg Ser Gln Asp Asn Val Ser Ile Ile Ile Ala Asp Leu Gly Gln Thr
            340                 345                 350

Asp Trp Arg Asn Leu Pro Leu Gln Lys Gln Asn Ile Leu Leu Glu Phe
        355                 360                 365

Gly Gln Ala Ile Ala Thr Ile Gly Val Val Ser Leu Gly Ile Trp Leu
    370                 375                 380

Ser Ser Gln Leu Ser Leu
385                 390

<210>  11
<211>  399
<212>  PRT
<213>  Picea sitchensis

<400>  11

Met Glu Leu Leu Ser Leu Val Tyr Tyr Asn Thr Phe Met Pro Asn Cys
1               5                   10                  15

Ser Gly Val Cys Val Asn Lys Cys Thr Val Gln Arg Arg Ser Ser Met
            20                  25                  30

Glu Cys Arg Lys Arg Gly Gly Val Gln Val Ala Gly Val Ala Ala Arg
        35                  40                  45

Pro Val Gln Val Asp Leu Ser Ala Ser Asp Thr Ser Lys Glu Gly Ser
    50                  55                  60

Ile Lys Trp Gly Trp Val Gly Ile Gln Gly Pro Arg Thr Glu Met Glu
65                  70                  75                  80

Asp Ala Ile Val Leu Arg Val Asp Gly Leu Asn Asn Phe Thr Tyr Ala
                85                  90                  95

Ala Val Phe Asp Gly His Ala Gly Phe Ala Ser Ala Lys Phe Leu Lys
            100                 105                 110

Asp Glu Leu Tyr Lys Glu Cys Ala Ile Ala Leu Gln Asp Gly Leu Leu
        115                 120                 125

Leu Glu Ser Asn Asp Leu Gly Ala Thr Arg Glu Ala Leu Arg Lys Ala
    130                 135                 140

Phe Ile Glu Thr Asp Arg Lys Leu Ile Ser Trp Leu Glu Glu Val Asn
145                 150                 155                 160

Asp Gly Val Asp Ser Gly Ser Thr Ala Thr Val Met Phe Val Gly Gly
                165                 170                 175

Gly Arg Leu Ile Ile Ser His Ile Gly Asp Ser Ser Val Val Leu Ser
            180                 185                 190

Arg Ser Gly Lys Pro Met Glu Ile Thr Ser Ala His Arg Pro Tyr Gly
        195                 200                 205

Asn Ser Lys Val Ser Leu Asp Glu Ile Arg Arg Ile Lys Ala Ala Gly
    210                 215                 220

Gly Trp Val Thr Asn Gly Arg Ile Cys Gly Asp Ile Ser Val Ser Arg
225                 230                 235                 240

Ala Phe Gly Asp Met Arg Phe Lys Thr Lys Met Lys Asp Met Leu Glu
                245                 250                 255

Glu Gly Ile Arg Glu Gly Lys Trp Thr Gly Lys Phe Ala Ser Arg Ile

260                 265                 270

Lys Leu Asn Gly Asp Trp Val Thr Ala Ile Pro Asp Ile Tyr His Ala
        275                 280                 285

Asp Leu Gly Pro Asp Ile Glu Phe Ile Ile Val Ala Ser Asp Gly Leu
    290                 295                 300

Trp Asp Cys Met Asn Ser Ser Asp Ala Val Lys Phe Val Arg Ser Gln
305                 310                 315                 320

Leu Arg Gln His Arg Asp Val Gln Cys Ala Cys Glu Ala Leu Ala Asn
                325                 330                 335

Ala Ala Leu Lys Arg Asn Thr Gln Asp Asn Val Ser Ile Ile Ile Ala
            340                 345                 350

Asp Phe Gly Arg Val Pro Gln Glu Asp Gly Pro Glu Lys Glu Gln Asn
        355                 360                 365

Phe Gly Ser Glu Ile Val Gln Ala Thr Ala Thr Val Gly Ile Val Thr
    370                 375                 380

Leu Gly Ile Trp Leu Ser Ser Leu Val Gly Gln Glu Leu Gly Arg
385                 390                 395

<210>  12
<211>  1167
<212>  DNA
<213>  Arabidopsis thaliana

<400>  12
atggcgcttc tgaggccgca tcttcatcgc tttcactcca acactctccg ccactcagcc      60

tatccttccg ccgacgctgg cggtggcctt gtcgtctatc ccacgtatgg tcgtcaccgt     120

tgctccgcga ttgcgatcga cgctccgtcg tctctgactg gcgtgacgcc gattcgttgg     180

ggttacacga gtgttcaagg attccgagat gagatggaag acgacatcgt aatccgttct     240

gatgctgtcg atagcttctc ctacgcagcg gtcttcgacg gccacgctgg gtcgtcctct     300

gtcaaattcc tcagggaaga gctgtataaa gagtgtgttg gtgcattgca agctggatcg     360

ttgttgaatg gaggcgattt cgcagcaatc aaggaagctt tgattaaggc ctttgaaagc     420

gttgaccgca atttgctcaa atggctagaa gctaacggtg acgaagaaga tgaatcaggt     480

tcaacagcca ctgtaatgat catcagaaat gatgtttcct tcattgctca tattggtgat     540

tcgtgtgcag tcttatctcg atctgggcaa atcgaggaat tgactgatta tcaccgtcca     600

tatggaagca gtagagcagc tattcaggaa gtgaaaaggg tcaaagaagc aggtggatgg     660

attgtcaatg gaaggatttg tggagacata gctgtatctc gtgcttttgg tgacatccgg 720

tttaagacaa agaagaatga tatgttaaag aagggtgttg atgaaggaag atggtccgag 780

aagtttgttt ccagaatcga gtttaaaggg gacatggttg tggcgactcc agatatcttc 840

caagtacctc tgacatctga tgtggaattc attattttag cttctgatgg attatgggac 900

tacatgaaga gctcagatgt ggttagttat gttagggatc agctccggaa acatgggaat 960

gtgcagcttg cttgtgaatc tcttgcgcaa gtagctttgg accggcgatc tcaggacaac 1020

attagcatca ttattgctga tctaggaaga acagagtgga agaatctccc cgcgcagcga 1080

caaaacgtag tggttgaact ggttcaggct gcaacgacta tcggtttagt tactgttggc 1140

atatggatgt cttcacatct atcttaa 1167

<210> 13
<211> 1161
<212> DNA
<213> Vitis vinifera

<400> 13
atggctttgc tgagccagca gttgcagaga ttctttctca cgacactcaa ctgtacatcc 60

aacttgaagg ccaccaagaa gaacaacagt gttgccctca aggagagggc aagatgctgc 120

tcagccatag ccattgatgc cccttctttt tcgggtgtgg cgggaatcag gtggggtttg 180

tgccgcctcc aagggcccag agaggagatg gaggacgagg ctgttgttcg gtctgatggc 240

cttgatgggt tctcctttgc cgcggttttt gacggtcatg ccggcttttc ttctgtcaag 300

ttcctcaggg atgaattgta caaagactgt gttgcagctc tgcaaggcgg gttgctcctg 360

agtgggaaaa atttcaacat aattcgagag gctttggaga aggcttttga aagtgctgat 420

gcaaaattat tgaattggct tgagacgact ggggaggatg tcgagtctgg ttcaacagcc 480

actgtgttgt tgattggaga cgatatggtg ttcatctcac atgttggtga ctcatgtgtg 540

gttctctctc gctctggaaa agcagaagaa ttgaccaatc ctcataggcc ttatggaagc 600

aacaaaagtt cgcttgaaga aatcagaaga atcagagaag caggtggatg gattgtcaat 660

ggaaggattt gcggagacat tgctgtgtcc cgctcttttg gtgacatgag gttcaagaca 720

aagaagaacg agatgctgga gaagggattg gaggaaggga gatggtccca aaaatttgtt 780

tctcgtgtac aattcactgg agacttggtc gttgcatctc ctgatgtttt ccaagtagct 840

cttggatcag atgcagaatt tttactgtta gcatctgatg gcttatggga ttatatgaac 900

agctcagaag cggtaacctt tgttaggaat gaacttcgac aacatggaga tgttcaggta 960

gcttctgaag cacttgcacg tgcagcactg gaccgacgca cacaagataa tgtcagcatc 1020

atcattgcag atttagggcg gacagactgg caaagtttgc cactgcagca acaaaattta 1080

tttctcgaat tggtgcaagc tcttggtacc attggcattg tgacgcttgg gatttggatg 1140

tcatctcagt ttagtttatg a 1161

<210> 14
<211> 1170
<212> DNA
<213> Oryza sativa

<400> 14
atggcgctgc tgagcccgcg tgtgccgcgg ctgccgctcg cctccgcctc cgccgctgga 60

gcggggctcc ggtgctgcgt cagcggcggc agggccgggt cggcggcgtg gtgccacgcg 120

tcggcggccg gcagcgtggc gtcgtcgtcg tcggagctgg aggccatccg gtgggggacc 180

gccaagctgc agggcgcgcg cgacgagatg gaggacgagg tcgtcctccg ccccggctcc 240

ctcctcgacg gcttctcctt cgccgccgtc ttcgacggcc acgccggatt ctccgccgtc 300

gagttcctca gggacgagct gtacaaggaa tgcgcggcgg cgttggacgg cggcgcggtg 360

ctgagcacca agaacctcga cgccatcacc gactcgatcc agcgcgcctt cgccaccgtc 420

gacgccaacc tctccacctg gctcgagcag atggacaagg aagatgagtc cggcgccacg 480

gccacggcca tgttcctcag gaacgatgtc cttgttgtct cgcacattgg cgactcctgc 540

ttgcaggtcg tatcacgcgg tgggaggcct caagctgtga ccaacttcca ccgaccgtac 600

gggaacaaga aagcgtctct tgaagaggtg aagcggatca gagcagcagg tggatggatt 660

gttgatggac gcatatgtgg agaaatttct gtgtctcgtg cttttggaga cattagattt 720

aagacacgga agaatgagat gttggtaaaa ggagttaaag aagggagatg gactgaaaag 780

tttatctcac gaataaattt taaaggagat ttaatagtct cttcacctga cgtatccctg 840

gtcgaacttg gaccagatgt ggaatttgtt ttgctagcaa cagatggtct ttgggattac 900

attaaaagct ctgaagcagt agcattagtt agggatcaac tacggcaaca tggggatgtc 960

caggttgcct gtgaggcact tggtcagata gctctggatc gtcggtcgca agacaacatt 1020

agtatagtca tagctgactt ggggaggaca aactggaagg agctgcctgc tcaaaggcca 1080

aatttattct tggagttaac tcaagcagtt gcaacagttg gggccgtctc attagggatt 1140

tatatttcat cgctccttgc gttgcagtag 1170

<210> 15
<211> 1182
<212> DNA
<213> Hordeum vulgare

<400> 15
gaattcggca cgaggctgag cccgcgtgtt ccgcggctgc cgctcgccgc tcactgcgcc 60

ggcgccggcg ccggcatcct ccggtgctgc ggccggcttg ctgcctccgc tgcgcgatgc 120

caagttacgg cgtcgggaag cgtggcggcg ggctcctcgt cgtcgtcgga gctggaggcc 180

atccggtggg gcagcgccaa actgcaggga gcccgcgagg agatggagga cgaggtcgtc 240

ctccgccccg agtccctcct ccacggcttc tcattcgccg ccgtcctcga cggccacgcc 300

ggtttctcca ccgtccagtt tctcagggac gagctgttca aggaatgcgc ggccgcgttg 360

gacggtggcg cggtgcttaa caccaagaat ctcgaggcca tcacggactc gatcaggcgt 420

gcctttgcca ccgtggacgc caacctctcc acctggcttc agcaaatgga taaggaagac 480

gactctggtg ctactgccac tgcccttttt ctgaggaatg atgtccttgt tgtctcgcac 540

attggtgact cttgcctggt catatcacgt ggtggaagac ctcagtctct gaccaatttt 600

catcgaccat acgggaacaa caaaacatcc cttgaagagg tcaagcggat tagggcagca 660

ggtggatgga ttcatgatgg acgcatatgt ggagacattt ctgtatctcg tgcttttgga 720

gacataagat ttaagacacg gaagaatgag atgctagtga aaggagttaa agaaggaaga 780

tggactgaaa agttcgtttc acgaataaaa tttaatgggg atctaataat ctcttcacct 840

gacgtatctc tggtagagct tggaccggat gcggaatttg ttttggtagc aactgatggt 900

ctttgggatt acattaaaag cactgaagct gtagcttttg tcagagatca actacgtcaa 960

catggagatg tccagcgggc ttgtgaggcg cttggggaga aagctctgga tcggcggtca 1020

caagacaata ttagcatagt catagctgac ttgggaagga caaactggca ggagttggcc 1080

gttccaaggc caaacgtgtt gttggagcta agtcaagcaa ttgcgactgt aggggctgta 1140

tcagtaggaa tttggatttc atggcttctc actttgcaat ag 1182

<210> 16
<211> 1388
<212> DNA
<213> Lycopersicon esculentum

<400> 16
cttacactaa cttcagtctg gtgagaacca agactcctcc atatttcatc aatggcctta 60

tctagtcccc acttgcaaaa agttctttta aacaaaattt gcagttacaa aattacatcc 120

aaaaagagca taatacagaa gcctcttgga ggaggtggag gtggaggtgg tgagggcaga 180

ggaagatgct gctctgcaat tgcaatcgac gcgccagctc catttaccag tgtttctggc 240

atcagatggg gttcaactat ggtccaaggt ccgcgtgaag agatggaaga tgatgctgtt 300

atcgttcaat cggatgatct tgatggcttc acttatgctg ctgtttttga tggccatgct 360

ggcttctcct ctgtcaagtt ccttagggaa gagctgtata aagaatgtgt tctagcttta 420

caaggagggc tgctattgaa cagaaaggac ctaaatgcaa tcaggaaggc actacaagaa 480

gcttttgaaa atgcggatag gaaactcttg aactggcttg agagtagcga gaaggaagat 540

gaatctggtg cgacagctac tgctctattt gttgggaatg atacattgat catcgcacat 600

gttggagatt catctgtggt gttgtcccga tctggaaaaa cagagatact aaccaattca 660

cacaggccgt atggaaacaa caaggtttct ctccaagaaa tcagaagaat aaatgaagca 720

ggtggatgga ttgtcaatgg aagaatttgt aatgatattt cagtatcccg tgcttttggt 780

gatatgagat ttaagacaaa gaagaaagag atgctggaga aaggggttaa agaacgcaga 840

tggtctgaaa agttcatttc acggattcag ttcaggggag acttggttat agcatctcca 900

gatgttttac aggtgaatct tggatcagat gccgaatttg tactattagc atctgatgga 960

ttatgggatt acatgaaaag cgatgaaata gtagatttcg ttaggaacca gcttcgagag 1020

catggtgatg ttcagatagc atgtgaagct cttgcactgt tagccttgga tcgacggaca 1080

caagataatg tcagcattgt cattgctgat ttggggagga cggattggcg aaatttacca 1140

gtaaagaaac agaatgttgt gttagaactg ggacaagctt tagtgacaat cagttttgtg 1200

tcaattggaa tatggctgtc aacaatgatt tccccttaga agtttgatag taatattagt 1260

taattgtata tagtattttt gtatgaaaag gaacataaag tttcaattat gccttgtaca 1320

tttacctgtc aaggtaatta agttatgtag cattttagat agtatattct aaaaaaaaaa 1380

aaaaaaaa 1388

<210> 17
<211> 1389
<212> DNA
<213> Zea mays

<400> 17
atccctccca actccagcgc cgctaggggt caaatcgaaa aatggcgttg ctgagcccgc 60

gcgtgccgcg gctgccgccc tccaccttca cctccgttgc cggtgcccgg tgctttggcc 120

ctgccgcgag gtgccaggcg acggcggcgg gcggcgtggc ggccgcgggg cccccgtcgt 180

cggagctgga ggccatccgg tggggcagcg ccaagctgca gggcgctcgc gacgagatgg 240

aggatgaggt cttgctccgc ccgggctccc tcctcgatgg cttctccttc gccgccgtct 300

tggatggaca cgccggcttc tccgccgtcc agttcctcag ggatgagctg tacaaggagt 360

gcgcagctgc gttggacggc ggcgcggtgc tcagcactaa gaatctcgac gctatcacgg 420

cctccatcca gcgtgccttc gccgctgtcg acgccaagct ctccacctgg ctcgagcaaa 480

cggacaagga cgactctggt gctacggcca ctgtcatgtt tctcaggagc gatgtgcttg 540

ttgtctcgca cattggggac tcttgcttgg tgatatcacg tggtggaaga tctgaagctt 600

tgactggctc tcatcgaccg tatgggaaca acaaaacgtc ccttgaagag gtcaagagga 660

tcagagcagc aggtggatgg attgttgatg ggcgcatatg tggagacata tctgtatctc 720

gtgcttttgg ggacataaga tttaagacac aaaagaatga aatgctggtg aaaggagtta 780

aacaaggaag gtggactgat aagttcatct cacgaataca atttaaagat gatatagtaa 840

tctcgtcacc tgatgtatct ctggtagagc ttggaccaga cgtggaattt gttctgttgg 900

caatggtctt tgggactaca taaaaagctc tgaagctgta gcttttgtca gagatcaact 960

ccgtcagcat ggtgatgtcc agttggcctg cgaggcactt ggtcagaaag ctctggatca 1020

acggtcccaa gacaatatta gcatagtcat agctgacttg gggaggacaa actggaaggc 1080

gttgcccgat gaaaggccga acttgttctt gtaactaagt caagcagtcg cgactgtagg 1140

ggttgtctca ataggaattt gggtctcgtc gttccttggg ttacagtaga actagctact 1200

ccgtccggat gtgtatatta cctactgagt ttacatcttt ctgctagcag tagcagctgc 1260

aaaatatatg tatattcgta tatagtgtgt gaattgatag ggtaatctgt ttgggtaata 1320

ctgagtgaac gtgtgaagta ctaaaaaaaa aaaaaaaaaa aaaaaaaaaa aacaaaaaaa 1380

aaaaaaaaa 1389

<210> 18
<211> 3120
<212> DNA
<213> Triticum aestivum

<400> 18
ggctgagccc gcgtgttccg cggctgccgc tcgccgccca ctccgccggc gccggcgccg 60

gatccctccg gtgctgcggc cgggttgctg cctccgctgc gcgatgccaa gtgacggcgt 120

cgggaagcgt ggcggcgggc tcctcgtcgt cgtcggagct ggaggccatc cggtggggca 180

gcgccaagct gcagggagct cgcgaggaga tggaggacga ggtcgtcctc cgccccgact 240

ccctcctcca cggcttctcc ttcgccgccg tcctcgacgg ccacgccggt ttctccaccg 300

tccagttcct gagggacgag ttgttcaagg aatgcgcggc cgcgttggac gggggcgcgg 360

tgcttaacac caagaatctc gaggccatca cggactcgat caggcgtgcc tttgccacca 420

tggacgccaa cctctccacc tggcttcagc aaatggataa ggaagacgac tctggtgcca 480

cggccacggc cttgtttctg aggaacgatg tccttgttgt ctcgcacatt ggtgactcct 540

gcttggtcat atcgcgtggt ggaagacctc aatctctgac aaattttcat cgaccatacg 600

ggaacaacaa aacatccctt gaagaggtca agcggattag ggcagcaggt ggatggattc 660

gtgatggacg cgtatgtgga gacatttctg tatctcgtgc ttttggagac ataagattta 720

aaacacggaa gaatgagttc gtctctccta ctccctgctc ttttacacac aattaatcac 780

tccatctgtc acattattcc caaataacat tcctgctatt tttaacatct cgtggtgtta 840

tgcaggatgc tagtaaaagg agttaacgaa ggaagatgga ctgaaaaatt cgtttcacgg 900

tatagcgctg aataaagtta tgttctttgt tattttctta actgcctgtg ttttcatttc 960

cttgtttttg tatcaataaa ttaaattctc aaacaaacca agttaggctt ttctgaaaca 1020

ataaggctat ttgttttagg agtgtttttg ctttcaatta tgtgccaaaa cttttttggg 1080

tggaatatgt ccaaaaacac gctctttcag tagtttctac tttcatgcaa agctgaaacc 1140

tttgtatact ggtttcatct ctaaaggggg ggactagcag ttgaatccgt atctattaag 1200

tgcacctact ttcaaacgac cttacatttt tttccactgt tgacaaatat aactgtgctc 1260

ccttttcaat tttttcctgg tggatgaaat ctaatgtttc cataatttgg cagatttgct 1320

tggaattttt tttggcttct atcttgaaca tgtaattgct cattattcaa aacctctctc 1380

taccatgctt gatagtagtt gctaattagc agctgaatcc gtatctatta agtgcagcta 1440

ctttccaacg accttccatt tttctccact gttgacaaat ataactgtgc tttttttttt 1500

cttttcctgg tggatgaaat gtagtgtttc caaaatttgg caggtttgct tgtttttttt 1560

ttgacttcta tacggctcat tgttcacaac ctctttcttg aagtttcaat aaaaaaaact 1620

cttgcttgaa ggtttgcacg agattttata atagctcatc gtttgtcgga ccacagatga 1680

tttgcttatc tgaggagagt aagctttctg ttgaaacatt cttttttcga tctgataaaa 1740

tattttatct gcaccttgag ttcttgttat aactggaatg atcattttat gatcagggat 1800

ttgatgaaaa ttttccatta ttatggttga aaactatcct gaattatata ttctcaatag 1860

ttatctgctt atccccacac atttaatgta tatccaactt ctgtacatgc ttttggttgc 1920

tactgcacag tatgagatga ctggtataat aagaaacgac atgtccaatt gtgtacctta 1980

atatcctaca acaaattaca cagataacat taccatgtta gatggtgaat attatagttt 2040

caaccataac tcttgccatg aaatatcatg tcgccaaagc tgagcctact gtggtttaca 2100

gcacttttgg tagatggtgt actgtacgtt aacttttttt gattatgcaa cctgttacgt 2160

gcagaataaa atttaaaggg gatctaataa tctcttcacc tgacgtatct ctggtggagc 2220

ttggaccgga tgtggaattt gttttggtag caaccgatgg tctttgggat tacattaaaa 2280

gcactgaagc tgtagcattt gtcagagatc aactatgtca acatggagat gtccaggtat 2340

gcagtctctc tctgcttaaa taaatgatac tcactatatg ccttttacag tacagtacat 2400

ttttgtatgc ttgctttttt ctcatttggt tgtttgtagc gggcctgtga ggcgcttggg 2460

gagaaagctc tggtattgtt gtttgccacc ttcatatctt ccattcattg tttcctttcc 2520

acctttatat tcggcgttta ttaaacctca ttacaataaa ctatgttctg attttccttt 2580

aaaaaaattg attacaggat cggcggtcac aagacaatat tagcatagtc atagctgact 2640

tggggaggac aaactggcag gagttgcccg ttccaacgcc aaacgtgttg ttggagctaa 2700

gtcaagcagt tgcgactgta ggggctgtat cagtaggaat ttggatttca tcgcttctca 2760

ctttgcaata ggcctagtta aggtcggggg tgtacatttt cacatcttcg gctccagctg 2820

caaatatata tgtatatcct tataggtaat tagtttcctg attaaactgt tcagtggtat 2880

ctggactgat gaaaagaaga agaaaaactt gtgctccagc tgctatctgg cagcgtacta 2940

ggatagggac ctgtgagacg cccgtctaaa ctttcctttt ctctttcccg ttttattggg 3000

ggatataaaa ccttatattt ggcacgcgac cggcaacatt tgataccatc actcctttct 3060

aaaatgaggc acttctaaat tttgcggcaa aaaaaaaaaa caaaaaaaaa aaaaaaacga 3120

<210> 19
<211> 1167
<212> DNA
<213> Sorghum bicolor

<400> 19
atggcgctgc tgagcccgcg cgtgccgcgg ctgccgccct ccaccttcac cttcaccttc 60

gccgccgcgg ccggtgcccc gtgctttggc cgtgccgcga ggtgccaggc gacggcggcg 120

ggcggcgtgg cggccgcggg gcccccgtcg tcggagctgg aggccgtcca gtggggcagc 180

gccaagctgc agggcgttcg cgacgagatg gaggatgaga tcttgctccg cccgggctcc 240

ctcctcgacg gcttctcctt cgccgccgtc ttggatggac acgccggctt ctccgccgtc 300

cagttcctca gggacgagct gtacaaggag tgcgcagccg cgttggacgg cggcgcggtg 360

ctcagcacta agaacctcga cgctatcacg gtctccatcc agcgtgcctt cgccgctgtc 420

gacgccaagc tctccacctg gctcgagcaa gcggacaagg acgacgactc tggtgctacg 480

gccactgcca tgtttctcag gaacgatgtc cttgttgtct cgcacattgg ggactcttgc 540

ttggtgatat cacgcggtgg aagacctgaa gctttgacca gctctcatcg accatatggg 600

aacaacaaaa cgtcccttga agaggtcaag aggatcagag cagcaggtgg atggattgtt 660

gatgggcgca tatgtggaga catatctgta tctcgtgctt ttggggacat aagatttaag 720

acgcgaaaga atgaaatgct ggtgaaagga gttaaagagg gaaggtggac tgataagttc 780

atctcacgaa taaaatttaa agatgatcta ataatctcgt cacctgatgt atctctggta 840

gagcttggac cagatgtgga atttgttcta ttagcaactg atggtctttg ggattacata 900

aaaagctctg aagctgtagc ttttgtcaga gatcaactac gtcaacatgg tgatgtccag 960

ttggcctgtg aggcacttgg tcagaaagct ctggatcaac ggtcaaaaga caatattagc 1020

atagtcatag ctgacttggg gaggacaaat tggaaggcgt tgcccgatga aagaccaaac 1080

atgttcttgg aactaagtca agcagtcgcg actgtagggg ttgtctcaat aggaatttgg 1140

ttttcgtcgt tccttggatt acagtag 1167

<210> 20
<211> 1152
<212> DNA
<213> Populus balsamifera subsp. trichocarpa

<400> 20
atggcattgt ttaggccaca gttggagaga ttcctatcaa ccaaactcca ttgtggccac 60

tccagtacca aaacccccgc taaaaacttc ttttttgcta ctaggcctaa aagccagtgt 120

tctgctatag caatagatgc cccatcttct ttaactgatg ttgctgggat ccggtggggt 180

tctgcaagct tgcaaggcgc acgtgaagag atggaggatg acatcataat tcgatcagat 240

ggccttgaag gattctcctt tgctgctgtt tttgatggcc atgctggttt ctcttctgtg 300

aagttcctca gggatgagtt gtacaaggag tgtgtagctg ctttacaagg cgggttgcta 360

ttgaatggaa aagatttcaa tgctattaga aaagcattag aagaggcttt tgaaagggtt 420

gatgcaaaat tgctagattg gtatgcctgt agagatggtg tgcaagacga atctggttcg 480

acagcgactg ctatgttcat tggaaacgaa aagctagtca tttcgcacat tggtgattca 540

tctgtggtcc tttctcgttc tggaaaaatg gaagtgttga ctgatgctca tcgaccttat 600

ggaagcaaca aaatctctct tcaagaaatc aaaagaatca gagaagcagg tggatggatt 660

gtcaatggaa ggatttgtgg agacatagcc gtatctcgtg cttttggtga catgcggttc 720

aagacaaaaa agaatgagat gctggagaaa ggagtcaagg aagggagatg gtctgaaaaa 780

tttagttctc gagtacaatt caatggggac ttggtcattg catctccaga agtttaccaa 840

atagcatttg gatcagatgc agagtttgta attctagcat ctgatggttt atgggattac 900

atgaacagct tagatgcagc tgcttttgtc aggaaccaac ttcaaaaaca tggagatgtt 960

cagctagctt gcgaagaact tgcacggaaa gctattgacc ggcggacaca agacaatgtt 1020

agcattatca ttgctgattt agggcggaca gactggcaaa atctgcctct ccagcaacaa 1080

aatgttgtgc ttgaactggg ccaagctttt gctaccattg gtattgtcac acttggaata 1140

tggatgtcat cc 1152

<210> 21
<211> 1173
<212> DNA
<213> Ricinus communis

<400> 21
atggcattgt tcagtcccca gttggagaga tttctattaa gcaagctcca ctatggcagc 60

tccaacttta aaaccactag taccaagaac ttgaacaact ttgttccagt gagaccaata 120

agtcaatgct ctgccattgc aatagacgca ccatcgtctc taactgatgt tgctggggtc 180

cgttgggggt ctgcgagctt gcagggcgca cgtgaagaga tggaagatta tattattgtt 240

cggtcagatg gccttgatgg gtactctttt gctggggttt ttgatggaca tggtggtatc 300

tcttctgtcg aattcctcag ggatgagctg tacaaggagt gtgttgcagc tttacaaggt 360

ggcttgctat tgagtggaaa agatttcaat gccacaagaa aagcattaac agaggctttt 420

gaaaatgtgg ataaaaagtt gttaaattgg cttgaaacaa ttggtgagga agatgaatca 480

ggttcaacag ccaccgttat gttcattgga aacaatatgc ttatcgtttc gcatattggt 540

gactcatgcc tggttctgtc tcgttctgga aaagctgaag ttttgactga atcccatcga 600

ccttatggga gcaacaaggt ttctcttcaa gaaatcaaaa gaatcaggga agcaggtggc 660

tggatcagca atggaagaat ctgtggagat atagctgtat cacgtgcttt cggagatata 720

aggttcaaga caaagaaaaa cgagatgctg cagaaaggag ttaaggaagg gagatggtct 780

gaaaaattta tttctagagt gcaattcaat ggggacttga tgactgcatc tccagatgta 840

tttcaagtag ctctgggttc agatgctgaa tttataatgt tagcatctga cggtttatgg 900

gattacatga acagctcaga tgcagtttct tttgtcagga accaacttcg gcaacatgga 960

gatgttcagc tagcttgcga agaacttgca caagcagctt tggatctacg ttcacaagac 1020

aatgttagca tcattattgc tgatttaggg caaacggatt ggcgaaattt gccactacag 1080

aaacaaaata tattgctcga gttcggccaa gctattgcca ccatcggtgt tgtgtcactt 1140

ggaatttggt tgtcatctca gctttcttta taa 1173

<210> 22
<211> 1200
<212> DNA
<213> Picea sitchensis

<400> 22
atggaattat taagcctcgt ttattataat actttcatgc caaattgttc aggagtttgt 60

gtgaacaaat gcacggttca gaggagaagt tccatggaat gcagaaaacg aggcggtgta 120

caggtggctg gagtagcagc gagacccgta caggtggatc tttcagcgtc tgacacgtca 180

aaagaaggaa gcattaaatg gggatgggtt ggaattcaag gccccagaac agagatggaa 240

gatgccatcg ttttacgagt ggacgggctt aataatttca cttacgcagc agtctttgac 300

ggccatgctg gattcgcttc tgctaaattt ttgaaggatg aattgtacaa agaatgtgca 360

atagctttac aagatggctt acttttggag tccaacgact tgggtgctac aagggaggca 420

ttaaggaaag cttttataga aactgatagg aagctgatat cctggctcga agaagtaaat 480

gatggggttg actcgggctc aacagcaact gttatgtttg taggaggtgg cagattgata 540

atttcacata ttggtgattc ttctgtggta ctttcccgtt caggaaagcc aatggagatt 600

acgagtgctc accgcccata tggaaatagc aaagtttccc ttgatgaaat acggcgtata 660

aaagcagcag gtggttgggt tacaaatggt cgaatttgtg gagacatatc tgtttcacgt 720

gcatttgggg atatgcgatt caagacaaag atgaaagata tgttggagga aggtattcga 780

gaagggaaat ggacagggaa atttgcatct agaatcaagc ttaatggtga ctgggttact 840

gccatacctg acatttatca tgcagatttg ggccctgata tcgagtttat tatagttgcc 900

tctgatggcc tttgggattg catgaacagt tcggatgcag taaagtttgt taggagtcaa 960

cttcgacaac atcgtgatgt gcagtgtgct tgtgaagcac ttgccaatgc tgctctgaag 1020

agaaatacac aggataatgt tagtattatc atcgcagact ttgggagagt tccccaggag 1080

gatggaccag agaaagagca aaattttgga agtgaaattg tgcaagctac tgcaacagtt 1140

ggtattgtaa ccttaggaat ttggctatca tctctagttg gccaagagct tggcagataa 1200

## Description

The invention relates to methods of increasing the photosynthesis rate of a plant cell and/or the production of biomass in a plant, as well as to plant cells and plants with increased photosynthesis rate/production of biomass. The present invention also relates to a phosphatase of the light-harvesting complex of photosystem II, to nucleic acids coding for such phosphatase, and to mutants of such nucleic acids. Moreover, the present invention relates to plant cells and plants, wherein the activity of said phosphatase is inhibited; in particular the present invention relates to plant cells and plants comprising the mutant nucleic acids.

A growing world population and a simultaneous reduction in farmland due to various factors (e.g. salinization) lead to aggrevating problems of world-wide food shortage. Similarly, ongoing climate changes require a continuous development of improved varieties of agricultural crops that are adapted to the new climate conditions (for example by increased tolerance to salt).

Traditionally, such improvements have been achieved by breeding, i.e. by introduction of the desired traits by crossbreeding and selection. In the wake of time and cost reduction, approaches based on genetic engineering are of increasing importance. This involves the targeted alteration of individual genes or proteins or even complete biosynthetic pathways, in order to obtain the desired improved characteristics. Frequently, an enhanced growth rate and thus an increase in the production of biomass is a designated goal in various areas of agricultural production, especially in the holistic utilization of plants (e.g. tree/wood production; sugarcane/sugar production, PHB (polyhydroxybutyrate, a bioplastic); vegetable plants/food production, plantibodies, oral vaccines; etc.). Indirectly, an enhanced growth rate can also lead to an increase in the production, e.g. in the case of grains. "Growth rate" and "biomass production" are controlled by multiple genes, which are very hard to identify for the purpose of genetic engineering.

A significant part of agricultural food production nowadays happens in large-scale greenhouses under controlled growth conditions (light, temperature, humidity). Due to changes in climate that may be imminent, intensified use of transgenic agricultural crops coupled with a still low acceptance of such plants in the public, an increase in the area of closed off greenhouse facilities is expected. An increased production of biomass under conditions of low light (reduction of energy cost) is therefore likely to become more and more important.

Of critical importance for the efficient production of biomass through a plant is the availability of energy carriers (ATP/reduction equivalents) and carbohydrates. These compounds are the products of photosynthesis, which therefore plays a central role in biomass production.

Photosynthesis is a process by which solar energy is converted into chemical bond energy. The overall reaction of photosynthesis is the light-driven conversion of carbon dioxide and water to glucose and oxygen:

6 C0₂ + 6 H₂O → C₆H₁₂O₆ + 6 O₂

The glucose is then used as a source of chemical bond energy as well as for the formation of various compounds that are essential for plant cell metabolism, and thus for plant survival and growth.

Photosynthesis is observed in plants as well as some protists, bacteria, and blue-green algae. The site of photosynthesis in plants is the chloroplast, a subcellular organelle that is found mainly in cells of plant leaves. Chloroplasts are cytoplasmic organelles surrounded by a double membrane. Enclosed by the inner membrane is the stroma, a soluble phase rich in enzymes. The stroma, in turn, contains a third membranous compartment, the thylakoids. These are flattened, sac-like membrane structures often forming stacks called grana.

Photosynthesis comprises two stages called the light reactions and the dark reactions. The light reactions require the presence of light, while the dark reactions do not depend on direct light exposure (nevertheless in most plants the dark reactions happen during the day). The thylakoid membranes are the site of the light reactions, whereas the dark reactions take place in the stroma of the chloroplast. In the light reactions, sunlight is absorbed and drives an electron transport chain that results in the formation of the energy carriers ATP and NADPH, forming O₂ as a by-product. In the dark reactions, a reaction driven by NADPH and ATP reduces CO₂ to glucose.

As the first step of the light reactions, energy from sunlight is absorbed by the green organic pigment chlorophyll in the thylakoid membrane and energizes an electron. Large multiprotein complexes called photosystems then allow, in collaboration with other components (such as the cytochrome b₆/f complex and the mobile components plastoquinone, plastocyanin, and ferredoxin), the conversion of the solar energy captured in excited chlorophyll molecules into chemical bond energy via an electron transport chain.

In general, photosystems are made up of two components: (1) a photochemical reaction center that consists of a complex of proteins and chlorophyll molecules and allows solar energy to be converted into chemical energy and (2) an antenna complex, which consists of a number of distinct membrane protein complexes (the light-harvesting complexes) that bind chlorophyll molecules and other pigments, which capture light energy and transfer it to the reaction center, resulting in excitation of the photosystem. In green plants, there are two photosystems called photosystem I and II (PSI and PSII).

Owing to their sessile life style, plants have to cope with environmental changes in their habitats, such as fluctuations in the incident light. Changes in light quantity or quality (i.e. spectral composition) result in imbalanced excitation of the two photosystems and decrease the efficiency of the photosynthetic light reactions. Plants can counteract such excitation imbalances within minutes by a mechanism called 'state transitions', which depends on the reversible association of the mobile pool of major light-harvesting (LHCII) proteins with photosystem II (state 1) or photosystem I (PSI) (state 2). In detail, the phosphorylation of LHCII, stimulated in low white light or by light of wavelengths specifically exciting PSII (red light), causes association of LHCII with PSI (state 2), thus directing additional excitation energy to PSI. Conditions like darkness or light of wavelengths specifically exciting PSI (far-' red light), as well as high intensities of white light, stimulate LHCII dephosphorylation and its migration to PSII (state 1), thus re-directing excitation energy to PSII.

The protein kinase responsible for phosphorylating LHCII is membrane-bound and activated upon reduction of the cytochrome *b*₆/*f*(Cyt *b*₆/*f*) complex via the plastoquinone (PQ) pool under state 2-promoting light conditions (low white light or red light). PQ oxidizing conditions induced by state 1-promoting light conditions (dark or far-red light) inactivate the LHCII kinase and result in association of LHCII with PSII (state 1). The LHCII kinase activity, however, is also inactivated under high white light conditions, when the stromal reduction state is very high. *In vitro* and, more recently, *in vivo* studies suggest that suppression of LHCII kinase activity might be mediated by reduced thioredoxin. In C. *reinhardtii* and *A. thaliana,* the orthologous thylakoid protein kinases Stt7 and STN7, respectively, are required for LHCII phosphorylation and state transitions. Co-immunoprecipitation assays showed that the Stt7 kinase interacts with Cyt *b₆*/*f,* PSI and LHCII, suggesting that Stt7 (and STN7 in *Arabidopsis)* directly phosphorylates LHCII, rather than being part of a Stt7/STN7-dependent phosphorylation cascade.

Under PQ oxidizing conditions when the LHCII kinase becomes inactivated, pLHCII is dephosphorylated by the action of a protein phosphatase, thus allowing the association of the mobile fraction of LHCII with PSII (state 1). Despite various attempts, however, nobody has been able to identify the responsible phosphatase.

To meet the increasing world food demands as well as for economic reasons, enhancing the rate of plant growth and hence of biomass production is becoming more and more important. To achieve this goal, it is of central importance to generate plant varieties with improved growth rate under suboptimal conditions, such as the conditions of low light found in some greenhouses.

Enhancing photosynthetic activity by fixing the photosynthetic machinery in one state would be an ideal approach to optimally adapt plants to specific conditions of light. For example, by shifting the balance of the phosphorylation state of LHCII completely to the side of the phosphorylated form (state 2), the photosynthesis rate of a plant at low light conditions could be optimized. One way to accomplish this would be to inactivate the phosphatase responsible for LHCII phosphorylation. However, so far there has been little success to increase the efficiency of photosynthesis by targeted gene technological approaches. In particular, as pointed out before nobody has succeeded in identifying the LHCII protein phosphatase(s) or discovering ways how to inhibit its activity.

An objective of the present invention was therefore to provide a method for increasing the photosynthetic activity of a plant cell.

Another objective of the present invention was to provide a plant in which the photosynthetic activity especially under low light conditions is increased.

The objects of the present invention are solved by a method of increasing the photosynthesis rate in a plant cell and/or the production of biomass in a plant, said method comprising:
inhibiting the activity of a phosphatase of the light harvesting complex of photosystem II, said phosphatase having an amino acid sequence which is
   a) SEQ ID NO 1:,
   b) at least 50 %, preferably at least 60 %, more preferably at least 70 %, even more preferably at least 80 %, even more preferably at least 90 %, and most preferably at least 99 % identical to SEQ ID NO: 1, or
   c) a homologue to SEQ ID NO: 1 from a plant species other than Arabidopsis thaliana, said homologue having an N-terminal chloroplast transit peptide (cTP), a C-terminal transmembrane domain and a protein phosphatase 2C signature, and said homologue having an amino acid sequence being selected fromSEQ ID NO:2-11.

In one embodiment said inhibiting the activity of said phosphatase occurs by one of the following:
(i) producing a mutant nucleic acid coding for a non-functional version of said phosphatase, through insertion, deletion or mutation of at least one nucleotide in the nucleic acid wildtype coding sequence of said phosphatase, and introducing said mutant nucleic acid into a plant cell,
(ii) producing a mutant nucleic acid coding for a version of said phosphatase having reduced expression, through insertion, deletion, mutation of at least one nucleotide at a position 1-500 nucleotides, preferably 1-100 nucleotides, upstream of the nucleic acid wildtype coding sequence of said phosphatase, and introducing said mutant nucleic acid into a plant cell,
(iii) producing a mutant nucleic acid by placing the nucleic acid coding for said phosphatase under the control of a regulatory sequence that switches off the expression of said nucleic acid coding for said phosphatase, and introducing said mutant nucleic acid into a plant cell,
(iv) producing, within a plant cell, a mutant nucleic acid coding for a non-functional version of said phosphatase or a version of said phosphatase having reduced expression, by exposing said plant cell to a mutagenic substance, such as ethyl methane sulfonate, or by exposing said plant cell to mutagenic radiation, such as UV-radiation or X-rays, and identifying the resultant mutant nucleic acid based on the recognition of mismatch hybridizations,
(v) introducing siRNA directed at the nucleic acid coding for said phosphatase, into a plant cell, or
(vi) blocking the activity of said phosphatase within a plant cell at the protein level by exposing said plant cell to a phosphatase inhibitor, such as NaF.

Exposing plant cells to mutagenic substances, such as ethyl methane sulfonate (EMS), or exposure to mutagenic radiation, leads to point mutations in the DNA sequence of TAP38 and therewith to its 'inactivation' without the introduction of transgenic material (as in the case of T-DNA insertions). These point mutations can be identified using, as an example, the TILLING-method ("targetting induced local lesions in genomes"), which is based on the recognition of mismatch-hybridisation via HPLC. Plants generated via such mutagenesis, e.g. EMS mutagenesis are by definition not genetically engineered and can be handled outside of 'S 1'-facilities.

In one embodiment increasing the photosynthesis rate and/or the production of biomass occurs under light exposure of said plant cell or plant to photosynthetic active radiation (PAR) in the range 50-200 µmol m⁻² S⁻¹, preferably 80-100 µmol m⁻²s⁻¹.

In one embodiment the nucleic acid coding for said phosphatase has a nucleic acid sequence selected from SEQ ID NO: 12-22.

In one embodiment the siRNA consists of 15 to 21, or 21 to 50, or 50 to 100, or 100 to 150, or 150 to 200 contiguous nucleotides of one of SEQ ID NO: 12-22, alone or in combination with its respective complementary counterpart strand.

In one embodiment said mutant nucleic acid is a mutant nucleic acid the expression of which in a plant cell results in at least 80 % less transcripts of said phosphatase, said mutant nucleic acid having an insertion, deletion or mutation of at least one nucleotide in the nucleic acid wildtype coding sequence of said phosphatase, or said mutant nucleic acid having an insertion, deletion or mutation of at least one nucleotide upstream of the nucleic acid wildtype coding sequence of said phosphatase.

In one embodiment said mutant nucleic acid has a nucleic acid sequence selected from SEQ ID NO: 12-22, into which or upstream of which a stretch of nucleotides has been inserted. Such stretch of nucleotides may, in the simplest case, consist of one nucleotide; it may, however, also consist of 10 nucleotides to 30000 nucleotides. This may, e.g., be achieved through the use of T-DNA which T-DNA may be derived from a number of bacteria, e.g. Agrobacterium tumefaciens. If the insertion is upstream of such coding sequence, it is preferably 1-500 nucleotides, more preferably 1-100 nucleotides, upstream of the start codon.

As an example, two mutant lines in A.thaliana that can be used in accordance with embodiments of the present invention carry T-DNA insertions 49 nucleotides (first mutant: *tap38-1*) and 77 nucleotides (second mutant: *tap38-2*) upstream of the Start-codon. A third T-DNA insertion line (third mutant: *tap38-3),* also in accordance with the present invention, carries an insertion in the 4^{th} exon. The exact insertion-sites are highlighted in the following wildtype full length cDNA and were confirmed by sequencing. The entire length of T-DNA at this gene locus was not sequenced. The nomenclature of the respective T-DNA lines as listed in databases is the following:

tap38-1(SAIL_514_C03); tap38-2 (SALK_025713); tap38-3 (GABI_232H12);

1 AGATATTTTT TTACGAAAAT CCA-(tap38-2)-CGGCCAC TAACCGTAAA GGACACTCTT
51 T-(tap38-1)-TGATTTTTC TCTTCTTCTA CTTAACTCGC TTTGCCTGAC TCACTGAGTC
101 ATGGCGCTTC TGAGGCCGCA TCTTCATCGC TTTCACTCCA ACACTCTCCG
151 CCACTCAGCC TATCCTTCCG CCGACGCTGG CGGTGGCCTT GTCGTCTATC
201 CCACGTATGG TCGTCACCGT TGCTCCGCGA TTGCGATCGA CGCTCCGTCG
251 TCTCTGACTG GCGTGACGCC GATTCGTTGG GGTTACACGA GTGTTCAAGG
301 ATTCCGAGAT GAGATGGAAG ACGACATCGT AATCCGTTCT GATGCTGTCG
351 ATAGCTTCTC CTACGCAGCG GTCTTCGACG GCCACGCTGG GTCGTCCTCT
401 GTCAAATTCC TCAGGGAAGA GCTGTATAAA GAGTGTGTTG GTGCATTGCA
451 AGCTGGATCG TTGTTGAATG GAGGCGATTT CGCAGCAATC AAGGAAGCTT
501 TGATTAAGGC CTTTGAAAGC GTTGACCGCA ATTTGCTCAA ATGGCTAGAA
551 GCTAACGGTG ACGAAGAAGA TGAATCAGGT TCAACAGCCA CTGTAATGAT
601 CATCAGAAAT GATGTTTCCT TCATTGCTCA TATTGGTGAT TCGTGTGCAG
651 TCTTATCTCG ATCTGGGCAA ATCGAGGAAT TGACTGAT-(tap38-3)-TA TCACCGTCCA
701 TATGGAAGCA GTAGAGCAGC TATTCAGGAA GTGAAAAGGG TCAAAGAAGC
751 AGGTGGATGG ATTGTCAATG GAAGGATTTG TGGAGACATA GCTGTATCTC
801 GTGCTTTTGG TGACATCCGG TTTAAGACAA AGAAGAATGA TATGTTAAAG
851 AAGGGTGTTG ATGAAGGAAG ATGGTCCGAG AAGTTTGTTT CCAGAATCGA
901 GTTTAAAGGG GACATGGTTG TGGCGACTCC AGATATCTTC CAAGTACCTC
951 TGACATCTGA TGTGGAATTC ATTATTTTAG CTTCTGATGG ATTATGGGAC
1001 TACATGAAGA GCTCAGATGT GGTTAGTTAT GTTAGGGATC AGCTCCGGAA
1051 ACATGGGAAT GTGCAGCTTG CTTGTGAATC TCTTGCGCAA GTAGCTTTGG
1101 ACCGGCGATC TCAGGACAAC ATTAGCATCA TTATTGCTGA TCTAGGAAGA
1151 ACAGAGTGGA AGAATCTCCC CGCGCAGCGA CAAAACGTAG TGGTTGAACT
1201 GGTTCAGGCT GCAACGACTA TCGGTTTAGT TACTGTTGGC ATATGGATGT
1251 CTTCACATCT ATCTTAAGTG AATTGAACAT TCTATCTCTT TCGTTAGTGT
1301 ATATAAAACC TGTAACTATG TCTAACACTC TAGTCTTCCC ATAGAAATGG
1351 AGATATGAAG ATACCTCACA AACAGAACCA GCTTTATATT ATTATATATC
1401 CCACTTTCAC AAGCGTTG

The objects of the present invention are also solved by a nucleic acid having a nucleic acid sequence encoding a phosphatase of the light-harvesting complex of photosystem II, said phosphatase having an amino acid sequence which is
(a) SEQ ID NO: 1,
(b) at least 50 %, preferably at least 60 %, more preferably at least 70 %, even more preferably at least 80 %, even more preferably at least 90 %, and most preferably at least 99 % identical to SEQ ID NO: 1, or
(c) a homologue to SEQ ID NO: 1 from a plant species other than Arabidopsis thaliana, said homologue having an N-terminal chloroplast transit peptide (cTP), a C-terminal transmembrane domain and a protein phosphatase 2C signature, and said homologue having an amino acid sequence being selected from SEQ ID NO: 2-11.

In one embodiment the nucleic acid according to the present invention has a nucleic acid sequence selected from SEQ ID NO: 12-22.

The objects of the present invention are also solved by a nucleic acid consisting of 15 to 21, or 21 to 50, or 50 to 100, or 100 to 150, or 150 to 200 contiguous nucleotides, preferably ribonucleotides, of the nucleic acid according to the present invention, alone or in combination with its complementary counterpart strand; in one embodiment, said nucleic acid consists of 15 to 21, or 21 to 50, or 50 to 100, or 100 to 150, or 150 to 200 contiguous nucleotides, preferably ribonucleotides, of one of SEQ ID NO: 12-22, alone or in combination with its respective complementary counterpart strand.

The objects of the present invention are also solved by a phosphatase of the light-harvesting complex of photosystem II, encoded by the nucleic acid according to the present invention.

The objects of the present invention are also solved by a mutant nucleic acid of the nucleic acid according to the present invention, the expression of which mutant nucleic acid in a plant cell results in at least 80 % less transcripts of phosphatase of the light-harvesting complex of photosystem II, said mutant nucleic acid having an insertion, deletion or mutation of at least one nucleotide in the nucleic acid sequence as defined above, or said mutant nucleic acid having an insertion, deletion or mutation of at least one nucleotide upstream of the nucleic acid sequence as defined above.

In one embodiment the mutant nucleic acid according to the present invention has a nucleic acid sequence selected from SEQ ID NO: 12-22, into which or upstream of which a stretch of nucleotides has been inserted. Such stretch of nucleotides may, in the simplest case, consist of one nucleotide; it may, however, also consist of 10 nucleotides to 30000 nucleotides. This may, e.g. be achieved through the use of T-DNA, which T-DNA may be derived from a number of bacteria, e.g. Agrobacterium tumefaciens. If the insertion is upstream of such coding sequence, it is preferably 1-500 nucleotides, more preferably 1-100 nucleotides, upstream of the start codon.

The objects of the present invention are also solved by a plant cell comprising a nucleic acid as defined above, and being selected from cells from Arabidopsis thaliana, Vitis vinifera, Oryza sativa, Hordeum vulgare, Lycopersicon esculentum, Zea mays, triticum aestivum, Sorghum, Populus trichocarpa, Ricinus communis and Picea sitchensis.

The objects of the present invention are also solved by a plant comprising one or several plant cells according to the present invention and being selected from Arabidopsis thaliana, Vitis vinifera, Oryza sativa, Hordeum vulgare, Lycopersicon esculentum, Zea mays, triticum aestivum, Sorghum, Populus trichocarpa, Ricinus communis and Picea sitchensis.

The inventors have recently identified a protein, namely a phosphatase of the light harvesting complex of photosystem II that is required for LHCII dephosphorylation and state transitions. This phosphatase is the long sought-after state transition phosphatase that dephosphorylates LHCII.

In plants without this phosphatase or having reduced activity of such phosphatase, LHCII is constitutively hyper-phosphorylated, and enhanced thylakoid electron flow is observed, indicative of an increased rate of photosynthesis. The increased photosynthesis, in turn, results in more rapid growth, in particular under low light regimes.

As used herein, the term "low-light conditions" refers to conditions with light exposure in the range 50-200 µmol m⁻² s⁻¹ µmol m⁻² s⁻¹ photosynthetic active radiation (PAR), preferably 80-100 µmol m⁻²s⁻¹ PAR.

As used herein, the term "photosynthetic active radiation", also abbreviated as "PAR", is meant to refer to the spectral range that photosynthetic organisms are able to use in the process of photosynthesis. Preferably such spectral range is in the range of from 400 nm to 700 nm.

A photosynthesis rate that is "increased" in accordance with the present invention is with reference to the photosynthesis rate of a plant cell/plant that has not been treated or selected in accordance with the present invention. Hence, as an example, the point of reference may, for example be a wild type plant cell/plant or a plant cell/plant that has not been treated with the appropriate siRNA. There are numerous ways to measure the photosynthesis rate in accordance with the present invention, and these measurements are known to a person skilled in the art. For example, such measurements include, for example, and without being limited thereto, the determination of the fraction of Q_{A} present in the reduced state and measurements of the maximum quantum yield (Fᵥ/F_{M}) and of the effective quantum yield (φ_{II}) of photosystem II. Such measurements are known to a person skilled in the art and can be performed without difficulties.

The inventors have also found that a plant cell and/or plant that has been treated in accordance with the method according to the present invention has an increased biomass. Hence, a plant cell or plant treated in accordance with the present invention has an increased biomass, with reference to a plant cell/plant that has not been treated by a method according to the present invention.

Sometimes herein reference is made to a sequence a which is "at least x% identical" to a sequence b. This is meant to refer to a scenario, wherein at least x% of the residues (nucleotides, amino acids) of sequence b are also present in sequence a in corresponding positions.

Moreover, sometimes reference is made herein to a "homologue" to a sequence c. This is meant to refer to a nucleic acid or protein that is from another species than sequence c and that has the same physiological function in said other species. More specifically, a homologue to the phosphatase of the light-harvesting complex of photosystem II of *Arabidopsis thaliana* (SEQ ID NO: 1) is also a phosphatase of the light-harvesting complex of photosystem II, but of another species. Such homologue is typically characterized by the presence of an N-terminal chloroplast transit peptide (cTP), a C-terminal transmembrane domain and a protein phosphatase 2C signature. Examples of such phoshphatase homologues are the corresponding phosphatases from or Arabidopsis thaliana, Vitis vinifera, Oryza sativa, Hordeum vulgare, Lycopersicon esculentum, Zea mays, triticum aestivum, Sorghum, Populus trichocarpa, Ricinus communis and Picea sitchensis

"Chloroplast transit peptides" (cTP), as used herein, can be unambiguously determined by experimental proof by a person skilled in the art and can be predicted, based upon their sequence. The same applies to "transmembrane domains" as well as "protein phosphatase 2C signatures". There exist various prediction algorithms for the subcellular localisation of a protein within a cell. The same is true for the prediction of potential transmembrane domains.

"siRNA", as used herein, is meant to refer to small interfering RNA-molecules as known to someone skilled in the art. siRNA refers to, typically, double-stranded fragments of 15-21, 21-50, 50-100, 100-150, 150-200 ribonucleotides, with a few unpaired overhang bases on each end. These short double-stranded fragments are then separated into single strands and integrated into an active RISC complex. After integration into the RISC, siRNAs base pair to their target mRNA and induce cleavage of the mRNA, thereby preventing it from being used as a translation template. Usage of siRNAs is also envisaged in the context of the present invention to inhibit the activity of the aforementioned phosphatase of the light harvesting complex of photosystem II. The design of such siRNAs suitable for this purpose can be done by someone skilled in the art without undue experimentation.

The present invention also envisages mutants of the nucleic acid coding for the aforementioned phosphatase, which mutants are non-functional in that, either, the transcription of the nucleic acid coding sequence is switched off due to the presence of an additional regulatory sequence, or altering the coding sequence such that a non-functional transcript/protein is produced. These mutants can be introduced into a plant cell through standard techniques, such as transformation, shot gun approaches or agrobacterium tumefaciens mediated transformation. The transgenic plant cells thus obtained can then subsequently be regenerated into whole plants.

In the plant cells/plants in accordance with the present invention, the rate of photosynthesis is increased and/or the production of biomass is increased, both with reference to a plant cell/plant that has not been treated in accordance with the present invention.

Moreover, reference is made to the sequences in the enclosed sequence listing, wherein
SEQ ID NO: is the amino acid sequence of the phosphatase of the light harvesting complex of photosystem II in Arabidopsis thaliana,
SEQ ID NO:2 is the amino acid sequence fo the phosphatase of the light harvesting complex of photosystem II in Vitis vinifera (common grape vine).
SEQ ID NO:3 is the amino acid sequence of the phosphatase of the light harvesting complex of photosystem II in Oryza sativa (rice).
SEQ ID NO:4 is the amino acid sequence of the phosphatase of the light harvesting complex of photosystem II in Hordeum vulgare (barley).SEQ ID NO:5 is the amino acid sequence of the phosphatase of the light harvesting complex of photosystem II in Lycopersicon esculentum (tomato).
SEQ ID NO:6 is the amino acid sequence of the phosphatase of the light harvesting complex of photosystem II in Zea mays (corn).
SEQ ID NO:7 is the amino acid sequence of the phosphatase of the light harvesting complex of photosystem II in Triticum aestivum (wheat).
SEQ ID NO:8 is the amino acid sequence of the phosphatase of the light harvesting complex of photosystem II in Sorghum bicolor (Sorghum).
SEQ ID NO:9 is the amino acid sequence of the phosphatase of the light harvesting complex of photosystem II in Populus trichocarpa (cottonwood).
SEQ ID NO: 1 0 is the amino acid sequence of the phosphatase of the light harvesting complex of photosystem II in Ricinus communis (castor oil plant).
SEQ ID NO:11 is the amino acid sequence of the phosphatase of the light harvesting complex of photosystem II in Picea sitchensis (Sitka spruce).
SEQ ID NO: 12-22 are the corresponding nucleic acid wildtype coding sequences of SEQ ID NO: 1-11.

Moreover, reference is made to the figures, wherein
Figure 1 shows a comparison of the TAP38 sequence with those of related proteins (homologues) from higher plants and moss,
Figure 2 shows the subcellular localization of TAP38;
Figure 3 shows the expression of TAP38 in tap38mutant, TAP38 overexpressor (oe TAP38) and wildtype (WT) plants;
Figure 4 shows that TAP38 is required for state transition;
Figure 5 shows that levels of phosphorylation of the light harvesting complex of photosystem II correlate inversely with TAP38 concentrations;
Figure 6 shows a quantification of PSI-LHCI and PSI-LHCI-LHCII complexes under state 2 conditions; and
Figure 7 shows the growth characteristics and photosynthetic performance of tap38 mutant plants.

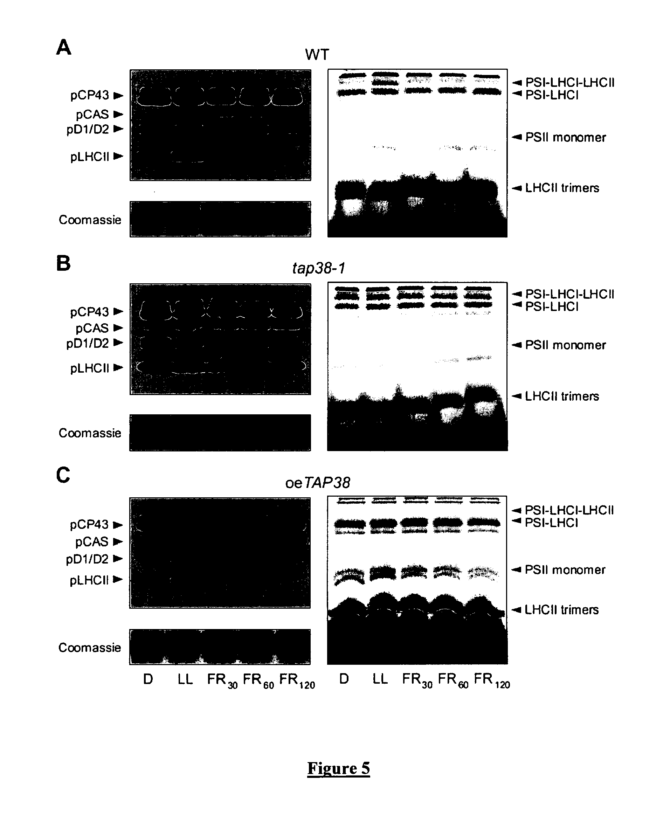

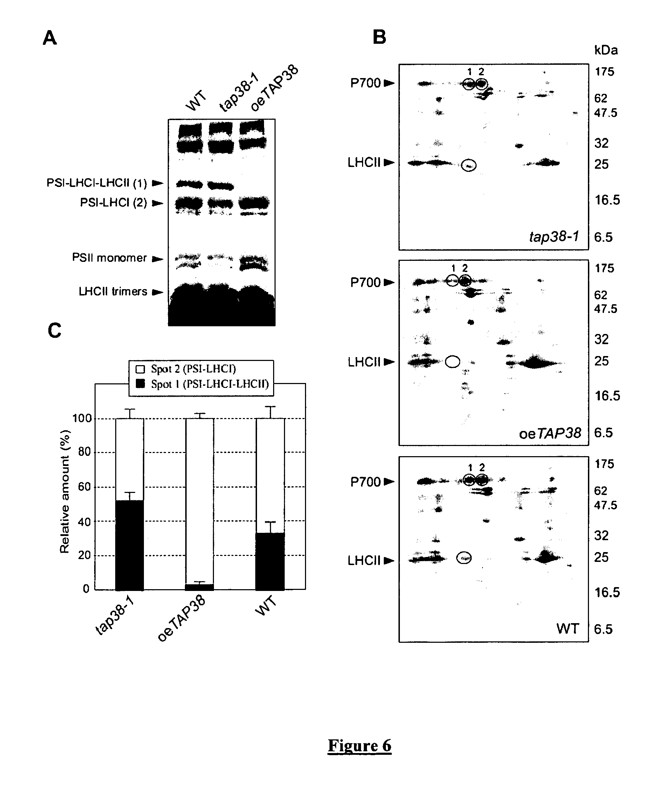

More specifically, the figures show the following:

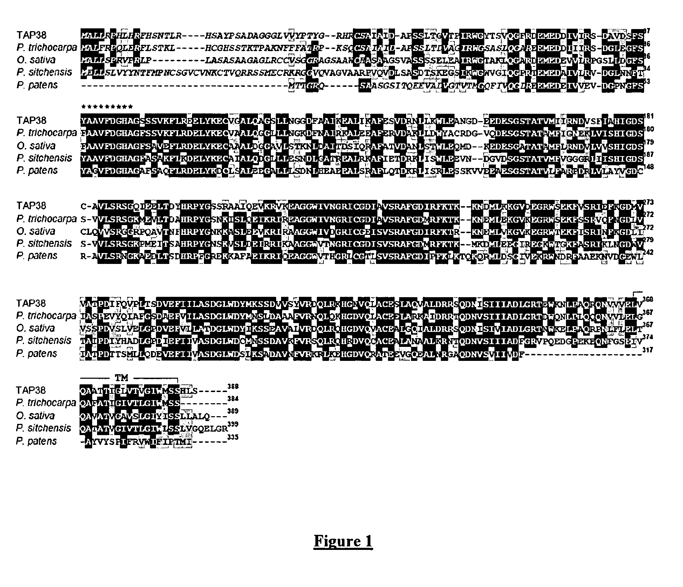

**Figure 1**. Comparison of the TAP38 sequence with those of related proteins from higher plants and moss.
   The amino acid sequence of the *Arabidopsis* TAP38 protein (At4g27800) was compared with related sequences from *Populus trichocarpa* (POPTRDRAFT_250893), *Oryza sativa* (Os01g0552300), *Picea sitchensis* (GenBank: EF676359.1) and *Physcomitrella patens* (PHYPADRAFT_113608). Black boxes highlight strictly conserved amino acids, and gray boxes closely related ones. Amino acids that constitute the protein phosphatase 2C signature are indicated by asterisks. Putative chloroplast transit peptides (cTPs) are indicated in italics, and the potential transmembrane domain (TM) is highlighted.

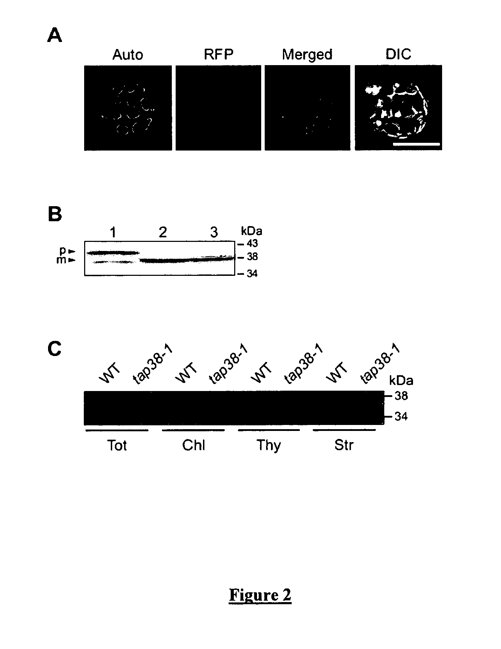

**Figure 2**. Subcellular localization of TAP38.
   (A) Full-length TAP38-RFP was transiently expressed in *Arabidopsis* protoplasts and visualized by fluorescence microscopy. Auto, chlorophyll autofluorescence; RFP, fusion protein; merged, overlay of the two signals; DIC, differential interference contrast image. Scale bar, 50 µm.
   (B) ³⁵S-labelled TAP38 protein, translated *in vitro* (lane 1, 10% translation product), was incubated with isolated chloroplasts (lane 2), which were subsequently treated with thermolysin to remove adhering precursor proteins (lane 3), prior to SDS-PAGE and autoradiography (p, precursor; m, mature protein).
   (C) Immunoblot analyses of proteins from WT and *tap38-1* leaves. Equal protein amounts were loaded. Tot, total protein; Chl, total chloroplasts; Thy, thylakoid proteins; Str, stromal proteins. Filters were immunolabelled with a TAP38-specific antibody.

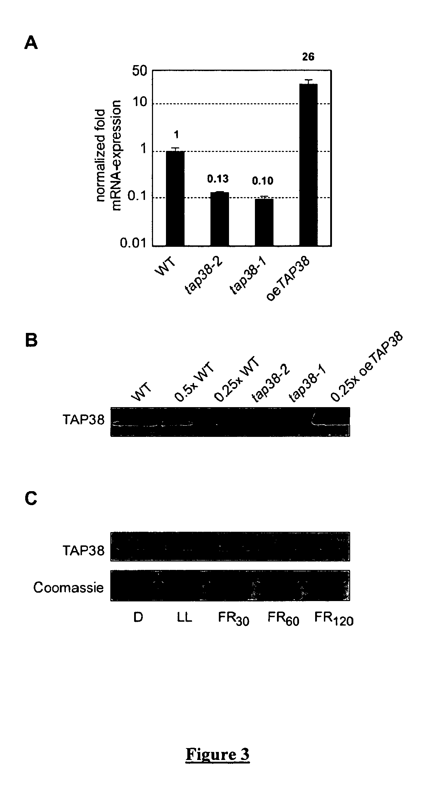

**Figure 3****.** Expression of TAP38 in *tap38* mutant, TAP38 overexpressor and WT plants.
   (A) Quantification of *TAP38* mRNAs by real-time PCR in WT, *tap38-1, tap38-2* and *oeTAP38* leaves using the primer combination 1-2 (as in Figures S1A and S1B).
   (B) Thylakoid proteins from WT and *tap38* mutants were loaded in the corresponding lanes. Reduced amounts of *oeTAP38* thylakoids, corresponding to 25% of WT amount were loaded in the lane marked as 0.25x *oeTAP38.* Additionally, decreasing levels of WT thylakoids were loaded in the lanes indicated as 0.5x WT and 0.25x WT. Filters were immunolabelled with a TAP38-specific antibody raised against the mature TAP38 protein.
   (C) Thylakoid membranes of WT plants exposed to different light conditions (see Figure 5) were separated by SDS-PAGE. Immunodecoration of the corresponding western was performed using a TAP38 specific antibody raised against the mature protein. A detail of a replicate gel, corresponding to the LHCII migration region, stained with Coomassie is shown as loading control.

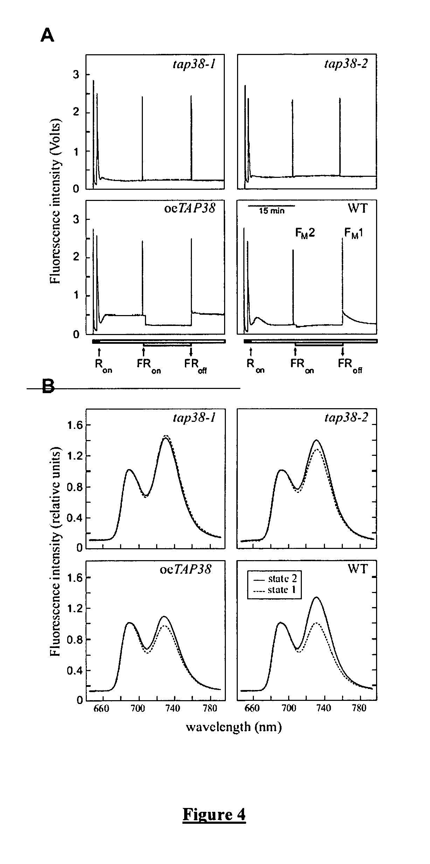

**Figure 4**. TAP38 is required for state transitions.
   (A) Red light (R) and red light supplemented with far-red (FR) light were used to induce transitions to state 2 and state 1, respectively. F_{M}1 and F_{M}2 represent maximal chlorophyll fluorescence levels in states 1 and 2, respectively. Horizontal bars indicate the length of illumination. Arrows point to the moment when the specific light is switched on/off. Traces are the average of 10 replicates.
   (B) Low-temperature (77K) fluorescence emission spectra of thylakoids were recorded after exposure of plants to light inducing either state 1 (dashed lines) or state 2 (solid lines) (see also Experimental Procedures). The excitation wavelength was 475 nm and spectra were normalized with reference to peak height at 685 nm. Traces are the average of 10 replicates.
F**igure 5.** Levels of LHCII phosphorylation correlate inversely with TAP38 concentrations. *Left panel,* Thylakoid proteins extracted from WT (A), *tap38-1* (B) and *oeTAP38* (C) plants kept in the dark (D, state 1), subsequently exposed to low light (LL, state 2), and then to far-red light for 30, 60 and 120 min (FR₃₀, FR₆₀, FR₁₂₀; state 1) were fractionated by SDS-PAGE. Phosphorylation of LHCII and PSII core proteins was detected by immunoblot analysis with a phosphothreonine-specific antibody. One out of three immunoblots for each genotype is shown
   *Right panel,* Thylakoid proteins of WT (A), *tap38-1* (B) and *oeTAP38* (C) plants treated as in the left panel were subjected to BN-PAGE analysis. Accumulation of the state 2 associated 670-kDa protein complex correlates with the phosphorylation level of LHCII. Note that *tap38-2* behaved very similarly to *tap38-1* (data not shown). One out of three BN-PAGEs for each genotype is shown
**Figure 6****.** Quantification of PSI-LHCI and PSI-LHCI-LHCII complexes under state 2 conditions.
   (A) BN PAGE of identical amounts of thylakoid proteins from WT, *tap38-1* and *oeTAP38* plants adapted to state 2 (low light; 80 µmol m⁻² s⁻¹). Bands representing the PSI-LHCI-LHCII (1) and PSI-LHCI (2) complexes are indicated.
   (B) The WT, *tap38-1* and *oeTAP38* lanes from the BN-PAGE in (A) were fractionated further by denaturing 2D PAGE. Gels were stained with Coomassie. P700, photosystem I reaction centre; LHCII, light-harvesting complex of PSII (the bands indicative for the PSI-LHCI-LHCII (1) and PSI-LHCI (2) complexes are encircled).
   (C) Densitometric quantification of the spots representing PSI-LHCI-LHCII (spot 1) and PSI-LHCI (spot 2) in panel B. Values are averages of three independent 2D gels for each genotype. Bars indicate standard deviations. Note that *tap38-2* behaves very similarly to *tap38-1* (data not shown).

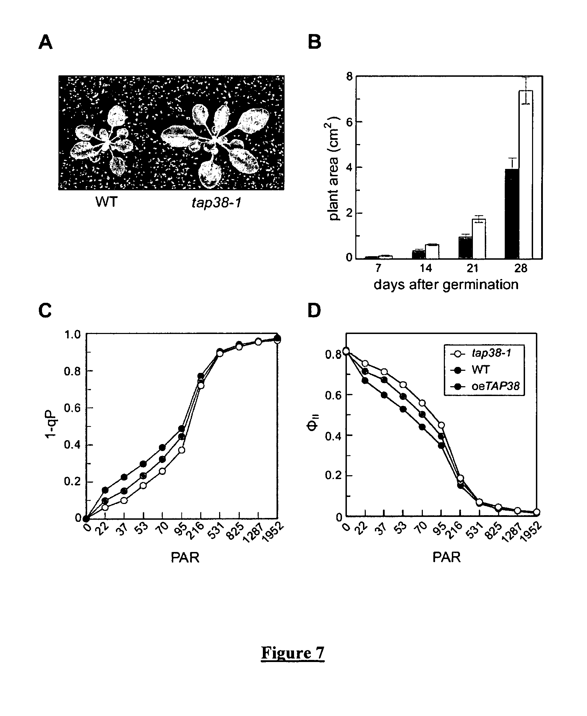

**Figure 7**. Growth characteristics and photosynthetic performance of *tap38* mutant plants.
   (A) Phenotypes of 4-week-old *tap38-1* and WT plants grown under low light conditions (80 µmol m⁻²s⁻¹) on a 12 h/12 h light/dark regime.
   (B) Growth curve. Leaf areas of twenty plants of each genotype (WT, grey bars; *tap83-1,* white bars) were measured over a period of four weeks after germination. Mean values ± standard deviations (SDs; bars) are shown.
   (C-D), Measurements of light dependence of the photosynthetic parameters 1-qP (C) and effective quantum yield of PSII (ΦII; D) of plants grown as in panel A. WT, filled grey circles; *tap38-1,* open circles; *oeTAP38,* filled black circles; PAR, photosynthetically active radiation in µmol m⁻² s⁻¹. Average values were determined from five independent measurements (SD <5%). Note that *tap38-2* and *tap38-1* plants behave similarly. However, the *tap38-2* mutant still synthesizes some TAP38 (see Figure 3B), and its phenotype is slightly less severe.

The invention will now be further described by the following examples, which are given to illustrate the invention, not to limit it.

### Examples

### Example 1

### Plant material and growth measurements

Procedures for plant propagation and growth measurements have been described elsewhere [44]. The *tap38-2* insertion line (SALK_025713) was identified in the SALK collection [34] (http://signal.salk.edu/), whereas insertion line *tap38-1* (SAIL_514_C03) originated from the Sail collection [33]. Both lines were identified by searching the insertion flanking database SIGNAL (http://signal.salk.edu/cgi-bin/tdna express). To generate *oeTAP38* lines the coding sequence of *TAP38* was cloned into the plant expression vector pH2GW7 (Invitrogen). For complementation of the *tap38-1* mutant the *TAP38* genomic DNA, together with 1 kb of its natural promoter was ligated into the plant expression vector pP001-VS. The constructs were used to transform flowers of Col-0 or *tap38-1* mutant plants by the floral dipping technique as described [45]. Transgenic plants, after selection for resistance to hygromycin *(oeTAP38)* or Basta herbicide (complemented *tap38-1 ),* were grown on soil in a climate chamber under controlled conditions (PAR: 80 µmol m⁻² s⁻¹, 12/12h dark/light cycles). The T2 generation of the *oeTAP38* plants was used for the experiments reported. Successful complementation of *tap38-1* mutants was confirmed by measurements of chlorophyll fluorescence and LHCII phosphorylation levels under light regimes promoting state transitions.

### Subcellular localization of the TAP38-dsRED fusion in Arabidopsis protoplasts

The full-length coding region of the *TAP38* gene was cloned into the vector pGJ1425, in frame with, and immediately upstream of the sequence encoding dsRED [32]. Isolation, transfection and fluorescence microscopy of *A. thaliana* protoplasts were performed as described [46].

### In-vitro import of TAP38 into pea chloroplasts

The coding region of *TAP38* was cloned into the pGEM-Teasy vector (Promega) downstream of its SP6 promoter region, and mRNA was *produced in vitro* using SP6 RNA polymerase (MBI Fermentas). The TAP38 precursor protein was synthesized in a Reticulocyte Extract System (Flexi®, Promega) in the presence of [³⁵S]methionine. Aliquots of the translation reaction were incubated with intact chloroplasts, and protein uptake was analysed after treatment of isolated chloroplasts with thermolysin (Calbiochem) as described previously [47]. Labelled proteins were subjected to SDS-PAGE and detected by phosphorimaging (Typhoon; Amersham Biosciences).

### cDNA synthesis, semi-quantitative reverse-transcriptase PCR and real-time PCR

Total RNA was extracted with the RNeasy Plant Mini Kit (QIAGEN) according to the manufacturer's instructions. cDNA was prepared from 1 µg of total RNA using the iScript cDNA Synthesis Kit (Bio-Rad) according to the manufacturer's instructions. For semi-quantitative reverse-transcriptase PCR, cDNA was diluted 10-fold, and 3 µl of the dilution was used in a 20-µl reaction. Thermal cycling consisted of an initial step at 95°C for 3 min, followed by 30 cycles of 10 s at 95°C, 30 s at 55°C and 10 s at 72°C. For real-time PCR analysis, 3 µl of the diluted cDNA was mixed with iQ SYBR Green Supermix (Bio-Rad). Thermal cycling consisted of an initial step at 95°C for 3 min, followed by 40 cycles of 10 s at 95°C, 30 s at 55°C and 10 s at 72°C, after which a melting curve was performed. Real-time PCR was monitored using the iQ5^{™} Multi Color Real-Time PCR Detection System (Bio-Rad). All reactions were performed in triplicate with at least two biological replicates.

### Protein isolation and immunoblot analysis

Total protein extracts and proteins from total chloroplasts, thylakoids and the stroma fraction were prepared from 4-week-old leaves in the presence of 10 mM NaF as described [46,48]. Immunoblot analyses with phosphothreonine-specific antibodies (Cell Signaling) or polyclonal antibodies raised against the mature TAP38 protein were performed as described [43].

### Blue-native (BN) PAGE and 2D polyacrylamide gel electrophoresis (2D PAGE)

For BN PAGE, thylakoid membranes were prepared as described above. Aliquots corresponding to 100 µg of chlorophyll were solubilised in solubilisation buffer (750 mM 6-aminocaproic acid; 5 mM EDTA, pH 7; 50 mM NaCl; 1.5% digitonin) for 1 h at 4°C. After centrifugation for 1 h at 21,000*g*, the solubilised material was fractionated by non-denaturing BN-PAGE at 4°C as described [37].

For 2D PAGE, samples were fractionated in the first dimension by BN-PAGE as described above and subsequently by denaturing sodium dodecyl sulfate PAGE as described previously [49]. Densitometric analysis of the stained gels was performed using the Lumi Analyst 3.0 (Boehringer, Mannheim, Germany).

### Measurements of state transitions and 77K fluorescence

State transitions were measured by pulse amplitude modulation fluorometry [35] and 77K fluorescence emission analysis [12,36]. For state transition measurements, five plants of each genotype were analysed, and mean values and standard deviations were calculated. *In-vivo* chlorophyll *a* fluorescence of single leaves was measured using the Dual-PAM 100 (Walz). Pulses (0.5 s) of red light (5000 µmol m⁻² s⁻¹) were used to determine the maximum fluorescence and the ratio (F_{M}-F₀)/F_{M} = Fᵥ/F_{M}. Quenching of chlorophyll fluorescence due to state transitions (qT) was determined by illuminating dark-adapted leaves with red light (35 µmol m⁻¹ s⁻¹, 15 min) and then measuring the maximum fluorescence in state 2 (F_{M}2). Next, state 1 was induced by adding far-red light (maximal light intensity corresponding to level 20 in the Dual-PAM setting, 15 min) and recording F_{M}1. qT was calculated as: qT = (F_{M}1-F_{M}2)/F_{M}2 [35].

For 77K fluorescence emission spectroscopy the fluorescence spectra of thylakoids were recorded after irradiating plants with light that favoured excitation of PSII (80 µmol m⁻² s⁻¹, 8 h) or PSI (LED light of 740 nm wavelength, 4.6 µmol m⁻² s⁻¹, 2 h). Thylakoids were isolated in the presence of 10 mM NaF as described [11], and 77 K fluorescence spectra were obtained by excitation at 475 nm using a Spex Fluorolog mod. 1 fluorometer (Spex Industries, Texas, USA). The emission between 600 and 800 nm was recorded, and spectra were normalized relative to peak height at 685 nm.

### Example 2

### Screening for candidate LHCII phosphatases

To identify the LHCII phosphatase, the present inventors systematically isolated loss-of-function mutants for known chloroplast protein phosphatases and assessed their capacity to dephosphorylate LHCII (see below for details). However, none of the nine protein phosphatases At3g52180 (DSP4/SEX4), At4g21210 (AtRPI), At1g07160, At3g30020, At4g33500, At1g67820, At2g30170, At3g10940, At4g03415, demonstrated to reside in the chloroplast [25-29], qualified as the LHCII phosphatase (data not shown). Next they extended their search to protein phosphatases tentatively identified as chloroplast proteins by proteomic analyses in *A. thaliana* [30,31]. Of those, At4g27800 turned out to be the most promising candidate.

### At4g27800 (TAP38) is a thylakoid-associated protein phosphatase

Proteins with high homology to At4g27800 exist in mosses and higher plants, but not in algae or prokaryotes. Furthermore, At4g27800 and its homologues share a predicted N-terminal chloroplast transit peptide (cTP), a putative transmembrane domain (TM) at their very C-terminus and a protein phosphatase 2C signature (Figure 1).

In protoplasts transfected with *At4g27800* fused to the coding sequence for the Red Fluorescent Protein (RFP) [32], the fusion protein localized to chloroplasts (Figure 2A). Chloroplast import assays with the radioactively labelled At4g27800 protein confirmed the uptake into the chloroplast with concomitant removal of its cTP. Mature At4g27800 has a molecular weight of ∼38 kDa (Figure 2B). Immunoblot analysis using a specific antibody raised against the mature At4g27800 protein (Figure 2C) detected the protein in thylakoid preparations but not in stromal fractions. At4g27800 was renamed TAP38 ('Thylakoid-Associated Phosphatase of 38 kDa').

### TAP38 expression in wild type, tap38 mutant and TAP38 overexpressor plants

Two *tap38* insertion mutants, *tap38-1* (SAIL_514_C03) [33]and *tap38-2* (SALK_025713) [34], were obtained from T-DNA insertion collections (Figure S1A). In *tap38-1* and *tap38-2* plants, amounts of *TAP38* transcripts were severely reduced -- to 10% and 13% of WT levels, respectively (Figure 3A). Conversely, in transgenic lines carrying the *TAP38* coding sequence under control of the 35S promoter of Cauliflower Mosaic Virus *(oeTAP38),* levels of *TAP38* mRNA were much higher than in wild type (WT) (Figure 3A). TAP38 protein concentrations reflected the abundance of *TAP38* transcripts: *tap38-1* and *tap38-2* thylakoids had <5% and ∼10% of WT levels, respectively, while the *oeTAP38* plants displayed >20-fold overexpression on the protein level (Figure 3B). TAP38 protein levels were also determined under light conditions relevant for state transitions (see Example 1). In WT plants, TAP38 was constitutively expressed at similar levels under all light conditions applied (Figure 3C).

### TAP38 is required for state transitions

To determine whether TAP38 is involved in state transitions, chlorophyll fluorescence was measured in WT, *tap38* and *oeTAP38* leaves (Figure 4A). Plants were exposed to light conditions that stimulate either state 2 (red light) or state 1 (far-red light) [35], and the corresponding maximum fluorescence F_{M}2 (state 2) and F_{M} 1 (state 1) values were determined. Because the light intensity chosen to induce state transitions did not elicit photoinhibition (as monitored by measurements of Fᵥ/F_{M} -- the maximum quantum yield; data not shown), changes in F_{M}, the maximum fluorescence, could be attributed to state transitions alone. This allowed us to calculate qT [35], the degree of quenching of chlorophyll fluorescence due to state transitions. In the *tap38* mutants, qT was markedly decreased *(tap38-1,* 0.01 ± 0.004; *tap38-2, 0.02* ± 0.004; WT, 0.11 ± 0.001). In *tap38-1* plants complemented with the *TAP38* genomic sequence (including its native promoter), qT values were normal (data not shown), confirming that state transitions require TAP38. Interestingly, *oeTAP38* plants exhibited qT values of about 0.01 ± 0.001, indicating that both absence and excess of TAP38 interfere with the ability to undergo normal state transitions.

To determine the antenna sizes of PSII and PSI, 77K fluorescence emission spectra were measured under state 1 (exposure to far-red light) and state 2 (low light) conditions as described [11,12,36] (Figure 4B). The spectra were normalized at 685 nm, the peak of PSII fluorescence. In WT, the transition from state 1 to state 2 was accompanied by a marked increase in relative PSI fluorescence at 730 nm, reflecting the redistribution of excitation energy from PSII to PSI. In contrast, in *tap38* leaves, the PSI fluorescence peak was relatively high even under state 1-promoting conditions, implying that the mutants were blocked in state 2 -- i.e. pLHCII should be predominantly attached to PSI. Additionally, under state 2-promoting light conditions, the PSI antenna size (expressed as F₇₃₀/F₆₈₅) was larger in *tap38* mutants than in WT *(tap38.1,* 1.47; *tap38.2,* 1.45; WT, 1.38; see also Table S1), arguing in favour of the idea that in *tap38* plants a larger fraction of the mobile pool of LHCII can attach to PSI. On the contrary, in *oeTAP38* plants the relative fluorescence of PSI hardly increased at all under conditions expected to induce the state 1→state 2 shift (Figure 4B; Table S1). This behaviour resembles that of *stn7* mutants, which are blocked in state 1, i.e. with LHCII permanently attached to PSII [12].

### Levels of LHCII phosphorylation correlate inversely with TAP38 concentrations

It is generally accepted that state transitions require reversible phosphorylation of LHCII [2,4,5]. Therefore, the phosphorylation state of LHCII was monitored under light conditions that favour state 1 (dark or far-red light treatment) or state 2 (low light). Plants with abnormal levels of TAP38 and WT plants were dark adapted for 16 h (state 1), then exposed to low light (80 µmol m⁻²s⁻¹, 8 h) (state 2), and then to far-red light (4.5 µmol m⁻² s⁻¹, 740 nm) for up to 120 min to induce a return to state 1. Thylakoid proteins were isolated after each treatment, fractionated by SDS-PAGE, and analysed with a phosphothreonine-specific antibody (Figure 5, left panels). WT plants showed the expected increase in phosphorylated LHCII (pLHCII) during the transition from state 1 (dark, D) to state 2 (low light, LL), followed by a progressive decrease in pLHCII upon exposure to far-red light (FR). In *tap38* mutants, levels of pLHCII were aberrantly high at all time points, while the *oeTAP38* plants again mimicked the *stn7* phenotype [9,12], displaying constitutively reduced levels of pLHCII.

### Quantification of PSI-LHCI-LHCII complex formation under varying TAP38 concentrations

To directly visualize how alterations in LHCII phosphorylation in lines lacking or overexpressing TAP38 affect the distribution of the mobile LHCII fraction between the two photosystems, we subjected thylakoid protein complexes of plants adapted to state 1 (dark and far-red light treatments) or state 2 (low light treatment) to non-denaturing Blue-native (BN) PAGE [37] (Figure 5, right panels). In this assay, a pigment-protein complex of about 670 kDa, which represents pLHCII associated with the PSI-LHCI complex, can be visualized [14,37,38]. Whereas in WT thylakoids the 670-kDa complex was only observable under state 2 conditions (Figure 5A, right panel), as previously reported [14,38], the constitutive phosphorylation of LHCII in the *tap38* mutants was associated with the presence of a prominent band for the 670-kDa complex under all light conditions (Figure 5B, right panel). The formation of the 670-kDa complex was totally prevented in *oeTAP38* plants with a block in state 1 and highly reduced levels of pLHCII (Figure 5C, right panel). Two-dimensional (2D) PA gel fractionation confirmed that the pigment-protein complex consists of PSI and LHCI subunits, together with a portion of pLHCII that associates with PSI upon state 1→ state 2 transition in WT plants (Figure 6B). Additionally, quantification of the different PSI complexes on 2D PA gels showed that the number of PSI complexes associated with LHCII was increased in the *tap38* mutants (Figures 6C), supporting the findings obtained from the 77K fluorescence analyses.

### Plants without TAP38 show improved photosynthesis and growth under low light

When kept under low light intensities (80 µmol m⁻² s⁻¹) that favour state 2, *tap38* mutants grew larger than WT plants (Figure 7A), whereas *oeTAP38* plants behaved like WT (data not shown). Detailed growth measurements revealed that the *tap38* mutants exhibited a constant growth advantage over WT plants, starting at the cotyledon stage (Figure 7B). Because this difference might be attributable to altered photosynthetic performance, parameters of thylakoid electron flow were measured. The fraction of Q_{A} (the primary electron acceptor of PSII) present in the reduced state (1-qP) was lower in *tap38-1* plants (0.06 + 0.01) than in WT (0.10 ± 0.01), when both genotypes were grown as in Figure 7A and chlorophyll fluorescence was excited with 22 µmol m⁻² s⁻¹ actinic red light. Comparable differences in the redox state of the primary electron acceptor persisted up to 95 µmol m⁻² s⁻¹ actinic red light (Figure 7C), indicating that the *tap38-1* mutant can redistribute a larger fraction of energy to PSI, in accordance with the increase in its antenna size under state 2 (see Figure 4B; Table S1 and Figure 6). This idea was supported by measurements of the maximum (Fᵥ/F_{M}) and effective (Φ_{II}) quantum yields of PSII. Fᵥ/F_{M} remained unaltered in mutant plants (see Figure 7D, dark-adapted plants, PAR = 0), indicating WT-like efficiency of mutant PSII complexes. However, Φ_{II} was increased in *tap38-1* (0.75 ± 0.01) relative to WT (0.72 ± 0.01), suggesting that electron flow through the thylakoids was more efficient in *tap38-1* (Figure 7D). The improvement in photosynthetic performance of the *tap38* mutants was most pronounced under low and moderate illumination (Figures 7C and 7D), as expected from their growth phenotype.

### Possible modes of action of TAP38

How does TAP38 control LHCII dephosphorylation? Three possibilities appear plausible: 1) TAP38 negatively regulates the activity of STN7 (e.g. by dephosphorylating it), 2) TAP38 dephosphorylates LHCII directly, or 3) forms part of a phosphorylation/dephosphorylation cascade that controls the activity of the LHCII kinase or phosphatase. The observation that *oeTAP38* plants, although showing a >20-fold increase in TAP38 levels, still exhibit residual LHCII phosphorylation (see Figure 5C), together with the fact that reversible phosphorylation of STN7 has not been demonstrated [39], strongly argues against the idea that TAP38 does inhibit STN7 by dephosphorylation. Differences in TAP38 levels resulted in a clear change in pLHCII levels: while in *tap38* mutants a strong reduction in TAP38 led to a constantly high level of pLHCII, strong overexpression of TAP38 *(oeTAP38)* severely reduced the amount of pLHCII. However, in WT where pLHCII levels can vary dramatically depending on the light conditions [9,12] (see also Figure 5A), TAP38 seems to be constitutively expressed under the different light conditions applied (see Figure 3C). A plausible explanation for this is that TAP38 is constitutively active and directly responsible for the dephosphorylation of LHCII. For that, TAP38 would need to be present in a certain concentration range (as it is the case for WT) to constantly dephosphorylate LHCII. In agreement with that, thylakoid protein phosphatase reactions have been described as redox-independent, leading to the conclusion that the redox dependency of LHCII phosphorylation is a property of the kinase reaction [40]. This, together with the observation that Stt7 levels increase under prolonged state 2 conditions (favouring LHCII phosphorylation) and decrease under state 1 conditions (favouring dephosphorylation of LHCII) [17], argues in favour of the hypothesis that the LHCII kinase is the decisive factor in controlling the phosphorylation state of LHCII. Despite the obvious TAP38 dosage dependence of LHCII dephosphorylation (see Figures 5 and 6), TAP38 activity could be regulated on other levels than only its abundance. However, the strong decrease or increase of TAP38 levels in *tap38* mutant and *oeTAP38* plants might interfere with other types of regulation in these genotypes.

### Is TAP38 the long-sought LHCII phosphatase?

As outlined above, the dependence of LHCII dephosphorylation upon TAP38 dosagewhen comparing *tap38* mutants, WT and TAP38 overexpressors -- strongly suggests that TAP38 dephosphorylates LHCII directly. Alternatively, TAP38 could act in a phosphorylation/dephosphorylation cascade that controls the activity of the LHCII phosphatase. Although the latter hypothesis cannot be excluded, a set of evidences point to a direct role of TAP38 on LHCII phosphorylation. Indeed, as in the case of STN kinases, extensive efforts searching to identify other LHCII phosphatase candidates failed: knout-out lines for all the protein phosphatases demonstrated to be located in the chloroplast [25-29] did not show any alteration in LHCII phosphorylation. Additionally, extensive biochemical studies did not reveal the existence of a complex network of phosphatases involved in LHCII dephosphorylation, but postulated the involvement of only two distinct chloroplast protein phosphatases from different families in the dephosphorylation of thylakoid phosphoproteins [20-23,41]. Our data support this notion, as shown by the absence of major alterations in the phosphorylation pattern of CP43, D1 and D2 subunits in *tap38* mutant plants (see Figure 5). Moreover, LHCII dephosphorylation was suggested to be catalysed by only two independent protein phosphatases, a membrane bound-one and a stromal protein phosphatase [41]. In contrast to this, our results clearly show that TAP38, a thylakoid-associated phosphatase, alone is responsible for LHCII dephosphorylation. Thus, although slightly leaky, the *tap38-1* mutants show a large fraction of LHCII in the phosphorylated state under all investigated conditions (see Figure 5). If a second LHCII phosphatase with redundant function would operate in chloroplasts, one would expect some residual dephosphorylation of LHCII. A plausible explanation for the previously shown stromal LHCII dephosphorylation activity [20] might be that during the preparation of stromal extracts a significant portion of TAP38 was released from the thylakoid membrane into the stroma.

Taken these observations together, it appears likely that, like in the case of the STN kinases, two distinct phosphatases are needed to dephosphorylate LHCII and PSII core proteins. TAP38, similar to the STN7 kinase, seems to have a high specificity for LHCII as substrate. The counterpart of STN8 [9,42] -- the PSII-core specific phosphatase, remains to be identified. However, as in the case of the STN7 and STN8 kinases, some degree of substrate overlap seems to exist also between the phosphatases, as shown by the more rapid dephosphorylation of D1/D2 subunits in the *TAP38* overexpressor lines exposed to far-red light conditions (see Figure 5C).

### Uncoupling of LHCII phosphorylation from PQ redox state

It is known that an increase in the relative size of the reduced fraction of the plastoquinone pool (PQH₂) enhances phosphorylation of LHCII [1,5,38,43]. Depletion of TAP38 in *tap38* mutants, however, increases both LHCII phosphorylation (see Figure 5B) and PQ oxidation (see 1-qP values in Figure 7C). This discrepancy can be resolved by assuming that the enhanced oxidation of PQ caused by the increase in PSI antenna size (and LHCII phosphorylation) in *tap38* plants is not sufficient to down-regulate the LHCII kinase to such an extent that it can compensate for the decline in LHCII dephosphorylation.

### How can absence of TAP38 improve photosynthesis and growth?

The enhanced photosynthetic performance indicated by an increase in Φ_{II} and a decrease of 1-qP (see Figure 7D), as well as the growth advantage of the *tap38* mutants under constant moderate light intensities that stimulate LHCII phosphorylation and state 2, can be attributed to the redistribution of a larger fraction of energy to PSI. This is in accordance with the increase in PSI antenna size in *tap38* mutants when compared to WT plants (see Figure 4B, Table S1 and Figure 6). Therefore, it is straightforward to speculate that the enhanced PSI antenna size provides the *tap38* mutants with a more robust photosynthetic electron flow under conditions that preferentially excite PSII and induce state 2. As a consequence of the more balanced light reaction, the photosynthetic efficiency is improved resulting in an increased growth rate. However, the fitness advantage will revert under conditions that induce state 1, or under more natural conditions with fluctuating light; here, it can be expected that *tap38* mutants perform less efficient than the WT with respect to photosynthesis and growth, very similar to what has been observed for the *stn7* mutant [12,13].

| **Table S1.** Energy distribution between PSI and PSII measured as the fluorescence emission ratio at 730 nm and 685 nm (F₇₃₀/F₆₈₅) | | | |
|---|---|---|---|
| | | **F₇₃₀/F₆₈₅** | |
| | **State 1** | | **State 2** |
| *WT* | 0.99 ± 0.05 | | 1.38 ± 0.03 |
| *tap38-1* | 1.49 ± 0.04 | | 1.47 ± 0.02 |
| *tap38-2* | 1.32 ± 0.03 | | 1.45 ± 0.04 |
| *oeTAP38* | 0.98 ± 0.02 | | 1.11 ± 0.03 |

The features of the present invention disclosed in the specification, the claims, and/or in the accompanying drawings may, both separately and in any combination thereof, be material for realizing the invention in various forms thereof.

### List of References

1. Allen JF, Forsberg J (2001) Molecular recognition in thylakoid structure and function. Trends Plant Sci 6: 317-326.
2. Eberhard S, Finazzi G, Wollman FA (2008) The dynamics of photosynthesis. Annu Rev Genet 42: 463-515.
3. Haldrup A, Jensen PE, Lunde C, Scheller HV (2001) Balance of power: a view of the mechanism of photosynthetic state transitions. Trends Plant Sci 6: 301-305.
4. Rochaix JD (2007) Role of thylakoid protein kinases in photosynthetic acclimation. FEBS Lett 581: 2768-2775.
5. Wollman FA (2001) State transitions reveal the dynamics and flexibility of the photosynthetic apparatus. EMBO J 20: 3623-3630.
6. Allen JF, Race HL (2002) Will the real LHC II kinase please step forward? Sci STKE 2002: PE43.
7. Allen JF (1992) Protein phosphorylation in regulation of photosynthesis. Biochim Biophys Acta 1098: 275-335.
8. Delosme R, Beal D, Joliot P (1994) Photoacoustic detection of flash-induced charge separation in photosynthetic systems - spectral dependence of the quantum yield. Biochim Biophys Acta 1185: 56-64.
9. Bonardi V, Pesaresi P, Becker T, Schleiff E, Wagner R, et al. (2005) Photosystem II core phosphorylation and photosynthetic acclimation require two different protein kinases. Nature 437: 1179-1182.
10. Lunde C, Jensen PE, Haldrup A, Knoetzel J, Scheller HV (2000) The PSI-H subunit of photosystem I is essential for state transitions in plant photosynthesis. Nature 408: 613-615.
11. Tikkanen M, Piippo M, Suorsa M, Sirpio S, Mulo P, et al. (2006) State transitions revisited-a buffering system for dynamic low light acclimation of Arabidopsis. Plant Mol Biol 62: 779-793.
12. Bellafiore S, Barneche F, Peltier G, Rochaix JD (2005) State transitions and light adaptation require chloroplast thylakoid protein kinase STN7. Nature 433: 892-895.
13. Frenkel M, Bellafiore S, Rochaix JD, Jansson S (2007) Hierarchy amongst photosynthetic acclimation responses for plant fitness. Physiol Plant 129: 455-459.
14. Pesaresi P, Hertle A, Pribil M, Kleine T, Wagner R, Strissel H, Ihnatowicz A, Bonardi V, Scharfenberg M, Schneider A, et al. (2009) Arabidopsis STN7 kinase provides a link between short- and long-term photosynthetic acclimation. Plant Cell (in press).
15. Vener AV, VanKan PJM, Rich PR, Ohad I, Andersson B (1997) Plastoquinol at the quinol oxidation site of reduced cytochrome bf mediates signal transduction between light and protein phosphorylation: Thylakoid protein kinase deactivation by a single-turnover flash. Proc Natl Acad Sci U S A 94: 1585-1590.
16. Zito F, Finazzi G, Delosme R, Nitschke W, Picot D, et al. (1999) The Qo site of cytochrome b6f complexes controls the activation of the LHCII kinase. EMBO J 18: 2961-2969.
17. Lemeille S, Willig A, Depege-Fargeix N, Delessert C, Bassi R, et al. (2009) Analysis of the chloroplast protein kinase Stt7 during state transitions. PLoS Biol 7: e45.
18. Rintamäki E, Martinsuo P, Pursiheimo S, Aro EM (2000) Cooperative regulation of light-harvesting complex II phosphorylation via the plastoquinol and ferredoxin-thioredoxin system in chloroplasts. Proc Natl Acad Sci U S A 97: 11644-11649.
19. Depege N, Bellafiore S, Rochaix JD (2003) Role of chloroplast protein kinase Stt7 in LHCII phosphorylation and state transition in Chlamydomonas. Science 299: 1572-1575.
20. Hammer MF, Sarath G, Markwell J (1995) Dephosphorylation of the thylakoid membrane light-harvesting complex-II by a stromal protein phosphatase. Photosynth Res 45: 195-201.
21. Hast T, Follmann H (1996) Identification of two thylakoid-associated phosphatases with protein phosphatase activity in chloroplasts of the soybean (Glycine max). J Photochem Photobiol B - Biol 36: 313-319.
22. Sun G, Bailey D, Jones MW, Markwell J (1989) Chloroplast thylakoid protein phosphatase is a membrane surface-associated activity. Plant Physiol 89: 238-243.
23. Vener AV, Rokka A, Fulgosi H, Andersson B, Herrmann RG (1999) A cyclophilin-regulated PP2A-like protein phosphatase in thylakoid membranes of plant chloroplasts. Biochemistry 38: 14955-14965.
24. Cohen P (1989) The structure and regulation of protein phosphatases. Annu Rev Biochem 58: 453-508.
25. Chastain CJ, Xu W, Parsley K, Sarath G, Hibberd JM, et al. (2008) The pyruvate, orthophosphate dikinase regulatory proteins of Arabidopsis possess a novel, unprecedented Ser/Thr protein kinase primary structure. Plant J 53: 854-863.
26. Kerk D, Conley TR, Rodriguez FA, Tran HT, Nimick M, et al. (2006) A chloroplast-localized dual-specificity protein phosphatase in Arabidopsis contains a phylogenetically dispersed and ancient carbohydrate-binding domain, which binds the polysaccharide starch. Plant J 46: 400-413.
27. Niittyla T, Comparot-Moss S, Lue WL, Messerli G, Trevisan M, et al. (2006) Similar protein phosphatases control starch metabolism in plants and glycogen metabolism in mammals. J Biol Chem 281: 11815-11818.
28. Schliebner I, Pribil M, Zuhlke J, Dietzmann A, Leister D (2008) A survey of chloroplast protein kinases and phosphatases in Arabidopsis thaliana. Curr Genomics 9: 184-190.
29. Sokolov LN, Dominguez-Solis JR, Allary AL, Buchanan BB, Luan S (2006) A redox-regulated chloroplast protein phosphatase binds to starch diurnally and functions in its accumulation. Proc Natl Acad Sci U S A 103: 9732-9737.
30. Sun Q, Zybailov B, Majeran W, Friso G, Olinares PD, et al. (2009) PPDB, the Plant Proteomics Database at Cornell. Nucleic Acids Res 37: D969-974.
31. Zybailov B, Rutschow H, Friso G, Rudella A, Emanuelsson O, et al. (2008) Sorting signals, N-terminal modifications and abundance of the chloroplast proteome. PLoS ONE 3: e1994.
32. Jach G, Binot E, Frings S, Luxa K, Schell J (2001) Use of red fluorescent protein from Discosoma sp. (dsRED) as a reporter for plant gene expression. Plant J 28: 483-491.
33. Sessions A, Burke E, Presting G, Aux G, McElver J, et al. (2002) A high-throughput Arabidopsis reverse genetics system. Plant Cell 14: 2985-2994.
34. Alonso JM, Stepanova AN, Leisse TJ, Kim CJ, Chen H, et al. (2003) Genome-wide insertional mutagenesis of Arabidopsis thaliana. Science 301: 653-657.
35. Jensen PE, Gilpin M, Knoetzel J, Scheller HV (2000) The PSI-K subunit of photosystem I is involved in the interaction between light-harvesting complex I and the photosystem I reaction center core. J Biol Chem 275: 24701-24708.
36. Tikkanen M, Nurmi M, Suorsa M, Danielsson R, Mamedov F, et al. (2008) Phosphorylation-dependent regulation of excitation energy distribution between the two photosystems in higher plants. Biochim Biophys Acta 1777: 425-432.
37. Heinemeyer J, Eubel H, Wehmhöner D, Jänsch L, Braun HP (2004) Proteomic approach to characterize the supramolecular organization of photosystems in higher plants. Phytochemistry 65: 1683-1692.
38. Pesaresi P, Lunde C, Jahns P, Tarantino D, Meurer J, et al. (2002) A stable LHCII-PSI aggregate and suppression of photosynthetic state transitions in the psael-1 mutant of Arabidopsis thaliana. Planta 215: 940-948.
39. Heazlewood JL, Durek P, Hummel J, Selbig J, Weckwerth W, et al. (2008) PhosPhAt: a database of phosphorylation sites in Arabidopsis thaliana and a plant-specific phosphorylation site predictor. Nucleic Acids Res 36: D1015-1021.
40. Silverstein T, Cheng L, Allen JF (1993) Chloroplast thylakoid protein phosphatase reactions are redox-independent and kinetically heterogeneous. FEBS Lett 334: 101-105.
41. Hammer MF, Sarath G, Osterman JC, Markwell J (1995) Assesing modulations of stromal and thylakoid light-harvesting complex-II phosphatase activites with phosphopeptide substrates. Photosynth Res 44: 107-115.
42. Vainonen JP, Hansson M, Vener AV (2005) STN8 protein kinase in Arabidopsis thaliana is specific in phosphorylation of photosystem II core proteins. J Biol Chem 280: 33679-33686.
43. Ihnatowicz A, Pesaresi P, Lohrig K, Wolters D, Muller B, et al. (2008) Impaired photosystem I oxidation induces STN7-dependent phosphorylation of the light-harvesting complex I protein Lhca4 in Arabidopsis thaliana. Planta 227: 717-722.
44. Leister D, Varotto C, Pesaresi P, Niwergall A, Salamini F (1999) Large-scale evaluation of plant growth in Arabidopsis thaliana by non-invasive image analysis. Plant Physiol Biochem 37: 671-678.
45. Clough SJ, Bent AF (1998) Floral dip: a simplified method for Agrobacterium-mediated transformation of Arabidopsis thaliana. Plant J 16: 735-743.
46. DalCorso G, Pesaresi P, Masiero S, Aseeva E, Schunemann D, et al. (2008) A complex containing PGRL1 and PGR5 is involved in the switch between linear and cyclic electron flow in Arabidopsis. Cell 132: 273-285.
47. Bölter B, Soll J (2007) Import of plastid precursor proteins into pea chloroplasts. Methods Mol Biol 390: 195-206.
48. Bassi R, dal Belin Peruffo A, Barbato R, Ghisi R (1985) Differences in chlorophyll-protein complexes and composition of polypeptides between thylakoids from bundle sheaths and mesophyll cells in maize. Eur J Biochem 146: 589-595.
49. Pesaresi P, Varotto C, Meurer J, Jahns P, Salamini F, et al. (2001) Knock-out of the plastid ribosomal protein L11 in Arabidopsis: effects on mRNA translation and photosynthesis. Plant J 27: 179-189.

## Claims

1. A method of increasing the photosynthesis rate in a plant cell and/or the production of biomass in a plant, said method comprising:
inhibiting the activity of a phosphatase of the light harvesting complex of photosystem II, said phosphatase having an amino acid sequence which is
a) SEQ ID NO:1,
b) at least 50 %, preferably at least 60 %, more preferably at least 70 %, even more preferably at least 80 %, even more preferably at least 90 %, and most preferably at least 99 % identical to SEQ ID NO: 1, or
c) a homologue to SEQ ID NO: 1 from a plant species other than Arabidopsis thaliana, said homologue having an N-terminal chloroplast transit peptide (cTP), a C-terminal transmembrane domain and a protein phosphatase 2C signature, and said homologue having an amino acid sequence being selected from SEQ ID NO: 2-11.

2. The method according to claim 1, wherein said inhibiting the activity of said phosphatase occurs by one of the following:
(i) producing a mutant nucleic acid coding for a non-functional version of said phosphatase, through insertion, deletion or mutation of at least one nucleotide in the nucleic acid wildtype coding sequence of said phosphatase, and introducing said mutant nucleic acid into a plant cell,
(ii) producing a mutant nucleic acid coding for a version of said phosphatase having reduced expression, through insertion, deletion, mutation of at least one nucleotide at a position 1-500 nucleotides, preferably 1-100 nucleotides upstream of the nucleic acid wildtype coding sequence of said phosphatase, and introducing said mutant nucleic acid into a plant cell,
(iii) producing a mutant nucleic acid by placing the nucleic acid coding for said phosphatase under the control of a regulatory sequence that switches off the expression of said nucleic acid coding for said phosphatase, and introducing said mutant nucleic acid into a plant cell,
(iv) producing, within a plant cell, a mutant nucleic acid coding for a non-functional version of said phosphatase or a version of said phosphatase having reduced expression, by exposing said plant cell to a mutagenic substance, such as ethyl methane sulfonate, or by exposing said plant cell to mutagenic radiation, such as UV-radiation or X-rays, and identifying the resultant mutant nucleic acid based on the recognition of mismatch hybridizations,
(v) introducing siRNA directed at the nucleic acid coding for said phosphatase, into a plant cell, or
(vi) blocking the activity of said phosphatase within a plant cell at the protein level by exposing said plant cell to a phosphatase inhibitor, such as NaF.

3. The method according to any of claims 1-2, wherein increasing the photosynthesis rate and/or the production of biomass occurs under light exposure of said plant cell or plant to photosynthetic active radiation (PAR) in the range 50-200 µmol m⁻² s⁻¹, preferably 80-100 µmol m⁻²s⁻¹.

4. The method according to any of claims 2-3, wherein the nucleic acid coding for said phosphatase has a nucleic acid sequence selected from SEQ ID NO: 12-22.

5. The method according to any of claims 2-4, wherein the siRNA consists of 15 to 21, or 21 to 50, or 50 to 100, or 100 to 150, or 150 to 200 contiguous nucleotides of SEQ ID NO: 12-22.

6. The method according to any of claims 2-5, wherein said mutant nucleic acid is a mutant nucleic acid the expression of which in a plant cell results in at least 80 % less transcripts of said phosphatase, said mutant nucleic acid having an insertion, deletion or mutation of at least one nucleotide in the nucleic acid wildtype coding sequence of said phosphatase, or said mutant nucleic acid having an insertion, deletion or mutation of at least one nucleotide upstream of the nucleic acid wildtype coding sequence of said phosphatase.

7. The method according to claim 6, wherein said mutant nucleic acid has a nucleic acid sequence selected from SEQ ID NO: 12-22 into which or upstream of which, preferably at a position 1-500 nucleotides, more preferably 1-100 nucleotides, upstream of which, at least one nucleotide has been inserted.

8. A nucleic acid having a nucleic acid sequence encoding a phosphatase of the light-harvesting complex of photosystem II, said phosphatase having an amino acid sequence which is
(a) SEQ ID NO: l,
(b) at least 50 %, preferably at least 60 %, more preferably at least 70 %, even more preferably at least 80 %, even more preferably at least 90 %, and most preferably at least 99 % identical to SEQ ID NO: 1, or
(c) a homologue to SEQ ID NO: 1 from a plant species other than Arabidopsis thaliana, said homologue having an N-terminal chloroplast transit peptide (cTP), a C-terminal transmembrane domain and a protein phosphatase 2C signature, and said homologue having an amino acid sequence being selected from SEQ ID N0:2-11.

9. The nucleic acid according to claim 8, having a nucleic acid sequence selected from SEQ ID NO: 12-22.

10. A nucleic acid consisting of 15 to 21, or 21 to 50, or 50 to 100, or 100 to 150, or 150 to 200 contiguous nucleotides, preferably ribonucleotides, of the nucleic acid according to any of claims 8-9, alone or in combination with its respective complementary counterpart strand.

11. A phosphatase of the light-harvesting complex of photosystem II, encoded by the nucleic acid according to any of claims 8-9.

12. A mutant nucleic acid of the nucleic acid as defined in any of claims 8-9, the expression of which mutant nucleic acid in a plant cell results in at least 80 % less transcripts of phosphatase of the light-harvesting complex of photosystem II, said mutant nucleic acid having an insertion, deletion or mutation of at least one nucleotide in the nucleic acid sequence as defined in any of claims 8-9, or said mutant nucleic acid having an insertion, deletion or mutation of at least one nucleotide upstream of the nucleic acid sequence as defined in any of claims 8-9.

13. The mutant nucleic acid according to claim 12, having a nucleic acid sequence selected from SEQ ID NO: 12-22 into which or upstream of which, preferably at a position 1-500 nucleotides, more preferably 1-100 nucleotides, upstream of which, at least one nucleotide has been inserted.

14. A plant cell comprising a nucleic acid as defined in any of claims 10, 12-13, and being selected from cells from Arabidopsis thaliana, Vitis vinifera, Oryza sativa, Hordeum vulgare, Lycopersicon esculentum, Zea mays, Triticum aestivum, Sorghum, Populus trichocarpa, Ricinus communis and Picea sitchensis.

15. A plant comprising one or several plant cells according to claim 14 and being selected from Arabidopsis thaliana, Vitis vinifera, Oryza sativa, Hordeum vulgare, Lycopersicon esculentum, Zea mays, Triticum aestivum, Sorghum, Populus trichocarpa, Ricinus communis and Picea sitchensis.
